# EUROPEAN PATENT APPLICATION

(11) **EP 4 382 023 A1**
(43) Date of publication of application: **12.06.2024**
(21) Application number: 22903352.7
(22) Date of filing: 05.12.2022
(51) Int. Cl.: A61B 1/005, A61B 1/00, A61B 1/012, A61B 1/12, A61B 1/04

(54) **ENDOSCOPE**

(30) Priority: 07.12.2021 CN 202111486328
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); TAN, Xiaofeng, Guangzhou, Guangdong 510000 (CN)
(74) Representative: Murgitroyd & Company
(86) International application number: PCT/CN2022/136449
(87) International publication number: WO 2023/103930

(57) **Abstract**

An endoscope, comprising a knob apparatus (200), a braking apparatus (300) and a flexible endoscope insertion tube (800). The knob apparatus (200) comprises a first wire guide wheel (210) and a second wire guide wheel (220) that are rotatably mounted inside a housing (100), as well as a first knob (230) and a second knob (240) that are located outside the housing (100). A first through hole (211) penetrates through the first wire guide wheel (210); the second wire guide wheel (220) passes through the first through hole (211) to extend out of the housing (100); the first knob (230) is sleeved on the first wire guide wheel (210); and the second knob (240) is arranged on the first knob (230) in a stacking manner and is sleeved on the second wire guide wheel (220). The braking apparatus (300) comprises a first braking assembly (310) and a second braking assembly (320). The first braking assembly (310) is arranged between the first knob (230) and the housing (100) and is used for braking the first knob (230) or the first wire guide wheel (210). The second braking assembly (320) is arranged on the side of the second knob (240) facing away from the first knob (230) and is used for braking the second knob (240) or the second wire guide wheel (220). With a more compact structure and more reasonable arrangement, the endoscope is more convenient to operate, helping to improve the reliability of operation.

## Description

### TECHNICAL FIELD

The present application relates to the technical field of medical devices, and in particular, to endoscopes.

### BACKGROUND

An endoscope is a detection instrument that integrates traditional optics, ergonomics, precision machinery, modern electronics, mathematics, software etc., and includes an image sensor, an optical lens, a lighting source, a mechanical device, etc. The endoscope is an important auxiliary medical device detecting organ lesions inside the human body, and is widely used in various surgical procedures. At present, most endoscope products are reusable products and require strict disinfection procedures after each use. A traditional endoscope includes a handle, an insertion tube, a bending section, a distal tip, and a main engine.

In order to expand the observation range, the bending section is controlled to bend over in four directions through a knob device to achieve four-way observation of the endoscope. However, the overall structural arrangement of the conventional endoscope is unreasonable. Limited by the structural design flaws of the endoscope, the operation is not convenient enough when controlling the bending of the bending section and the braking of the knob device.

### SUMMARY

Accordingly, it is necessary to provide an endoscope with reasonable structural arrangement and convenient operation.

An endoscope includes: a housing; a knob device; a braking device; and a bending section. The housing is provided with a first mounting hole that is in communication with inside and outside of the housing. The knob device includes a first pulley, a second pulley, a first knob and a second knob. The first pulley and the second pulley are rotatably mounted in the housing, and the first knob and the second knob are located outside the housing. The first pulley extends out of the housing through the first mounting hole, and a first through hole extends through the first pulley. The second pulley extends out of the housing through the first through hole. The first knob is sleeved on the first pulley, and the second knob is stacked on the first knob and sleeved on the second pulley. The braking device includes a first braking assembly and a second braking assembly. The first braking assembly is disposed between the first pulley and the housing, and configured to brake the first knob or the first pulley. The second braking assembly is disposed on a side of the second knob facing away from the first knob, and configured to brake the second knob or the second pulley. The bending section is located outside the housing, and respectively connected to the first pulley and the second pulley through a pulling wire.

When the above-mentioned endoscope is in use, the first knob is rotated to drive the first pulley to rotate in the housing; and after the first pulley is rotated, one side of the bending section is pulled by the pulling wire, so that the bending section is bent in an up-down direction. In order to achieve a fixation at a specific angle, the first pulley or the first knob is braked by the first braking assembly, and the pulling wire cannot be pulled to move, so that the bending section remains bent at a specific angle. Similarly, the second knob is rotated to drive the second pulley to rotate in the housing; after the second pulley is rotated, one side of the bending section is pulled by the pulling wire, so that the bending section is bent in a left-right direction. In order to achieve a fixation at a specific angle, the second pulley or the second knob is braked by the second braking assembly, and the pulling wire cannot be pulled to move, so that the bending section remains bent at a specific angle. In this way, through the first knob, the second knob, the first braking assembly, and the second braking assembly, the control of the bending of the bending section in four directions can be stably realized, thereby improving the stability of the operation. In addition, in terms of structural layout, the second pulley extends through the first pulley through the first through hole. In addition, the first knob and the second knob are stacked on the outside of the housing, and the first braking assembly is located between the first pulley and the housing, and the second braking assembly is disposed on the side of the second knob facing away from the first knob. Through such arrangement, the endoscope has more compact structure, and more reasonable arrangement. As such, during operation, it is convenient for the operator to accurately and quickly distinguish the bending in the up-down direction and the bending in the left-right direction of the bending section, which makes the operation more convenient, and is beneficial to the reliability of the operation.

In an embodiment, the first braking assembly includes: a first fixed frame; a first braking button; and at least two first braking members slidably disposed on the first fixed frame. The first fixed frame is positioned on the housing. The first braking button is sleeved outside the first fixed frame. When the first braking button is rotated, the first braking button is configured to push the first braking member to slide to hold the first knob or the first pulley tightly.

In an embodiment, the first fixed frame is provided with a first working chamber into which the first pulley extends. The first knob extends into the first working chamber and is sleeved on the first pulley. When the first braking button rotates, the first braking button is configured to push the first braking member to extend into the first working chamber and hold the first knob tightly.

In an embodiment, the knob device further includes a first bracket disposed in the housing. A second through hole extends through the second pulley. The first bracket extends through the second through hole and extends out of the housing. The second braking assembly is mounted on the first bracket.

In an embodiment, the second braking assembly includes: a second fixed frame; a second braking button; and at least two second braking members slidably disposed on the second fixed frame. The second fixed frame is positioned on the first bracket. The second braking button is sleeved outside the second fixed frame. When the second braking button rotates, the second braking button is configured to push the second braking member to slide to hold the second knob or the second pulley tightly.

In an embodiment, the first bracket is provided with a first fixing hole extending therethrough. The second braking button is provided with a second fixing hole corresponding to the first fixing hole. A fastener is inserted into the first fixing hole and the second fixing hole, so that the second braking button is rotatably mounted on the first bracket.

In an embodiment, the second knob is provided with a braking ring. The second fixed frame is provided with a second working chamber into which the braking ring extends. When the second braking button rotates, the second braking button is configured to push the second braking member to extend into the second working chamber and hold the brake ring tightly.

In an embodiment, the endoscope further includes: a gas-water valve; and a distal tip disposed on the bending section. The gas-water valve is embedded in the housing. A first coupling part is disposed on an inner wall of the housing. The gas-water valve is provided with a second coupling part that is engaged with the first coupling part.

In an embodiment, the endoscope further includes a negative pressure valve embedded in the housing. A third coupling part is disposed on an inner wall of the housing. The negative pressure valve is provided with a fourth coupling part that is engaged with the third coupling part.

In an embodiment, the endoscope further includes a three-way valve. The inner wall of the housing is provided with a fifth coupling part. The three-way valve is provided with a sixth coupling part that is engaged with the fifth coupling part. The three-way valve is provided with a first port, a second port and a third port that are in communication with one another. The first port is in communication with the negative pressure valve. The second port is configured to be in communication with a device channel valve. The third port is configured to be in communication with a device channel of the distal tip.

### BRIEF DESCRIPTION OF THE DRAWINGS

The drawings forming a part of this application are used to provide a further understanding of the present application. The illustrative embodiments of the present application and descriptions thereof are used to explain the present application and do not constitute an improper limitation of the present application.

In order to more clearly illustrate the technical solutions in the embodiments of the present application, the drawings needed to be used in the description of the embodiments will be briefly introduced below. Obviously, the drawings in the following description are only some embodiments of the present application. For those of ordinary skill in the art, other drawings can also be obtained based on these drawings without exerting creative efforts.

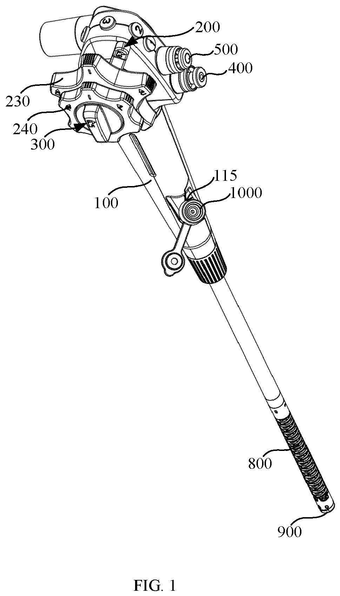

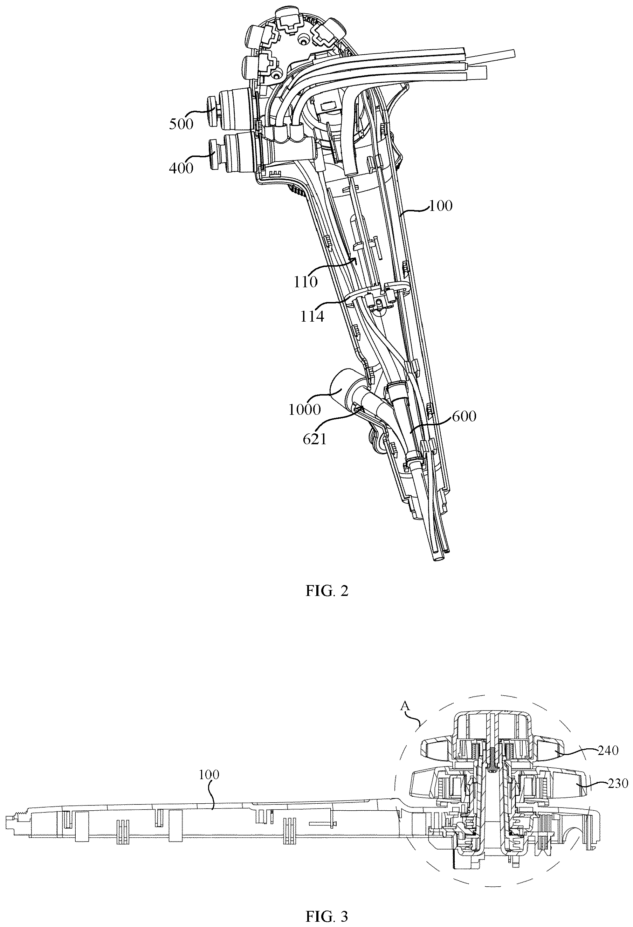

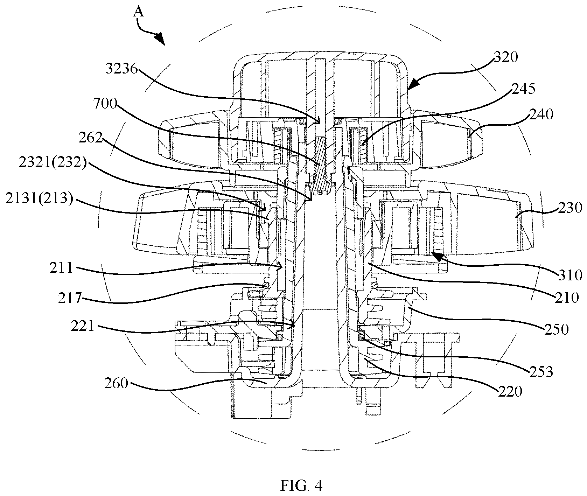

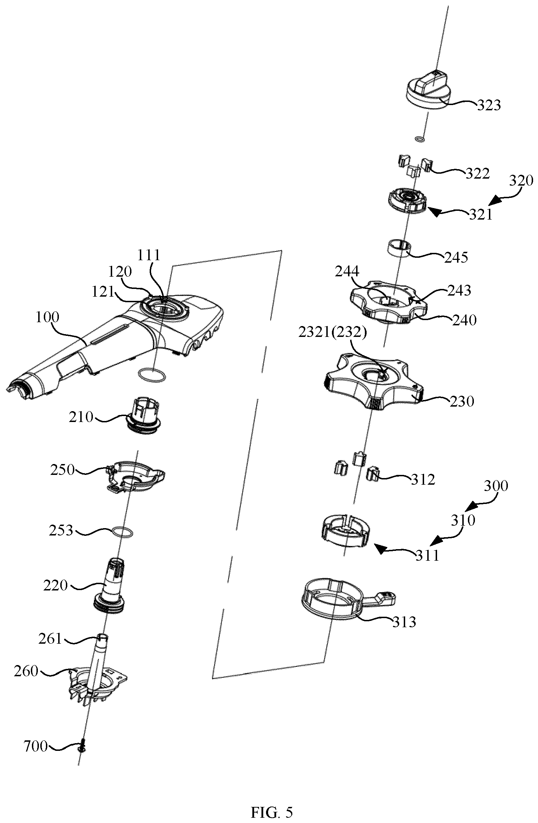

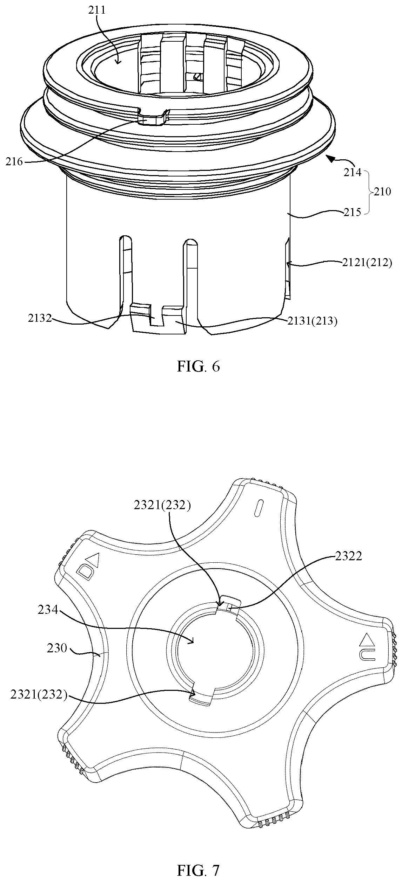

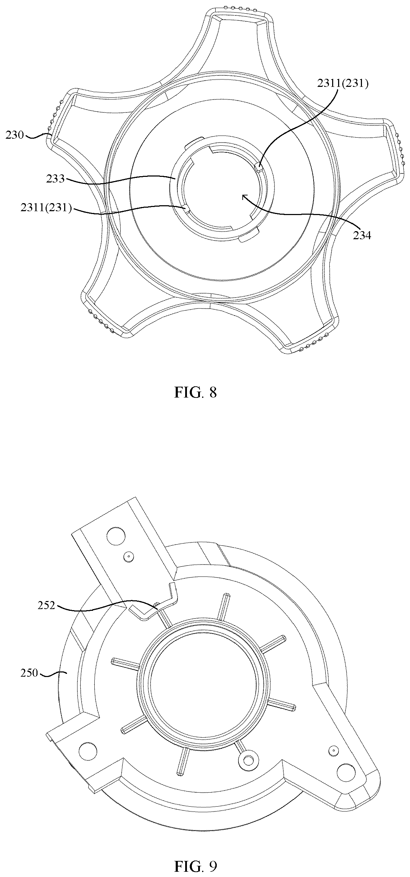

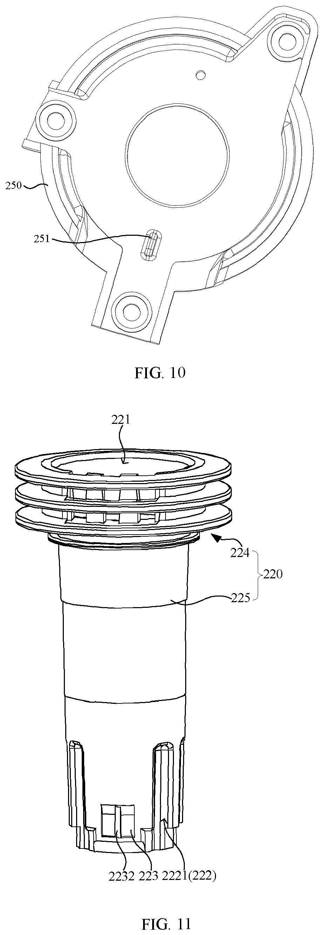

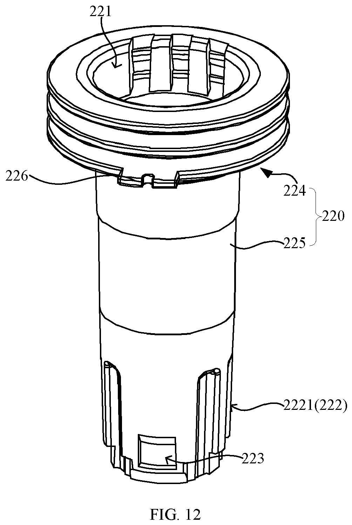

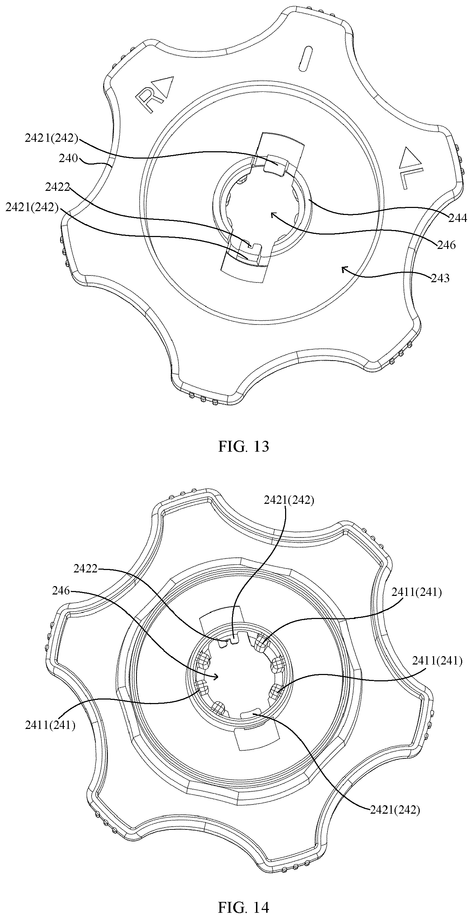

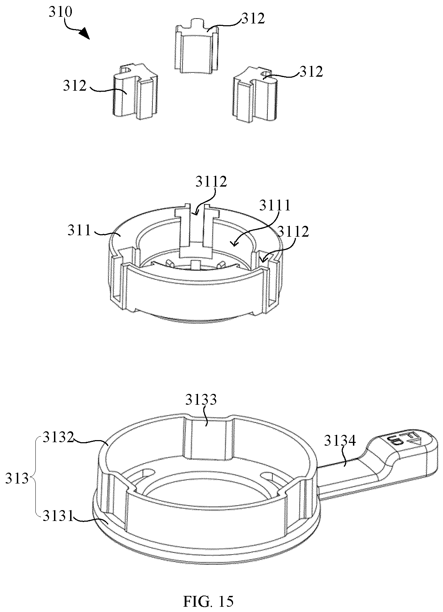

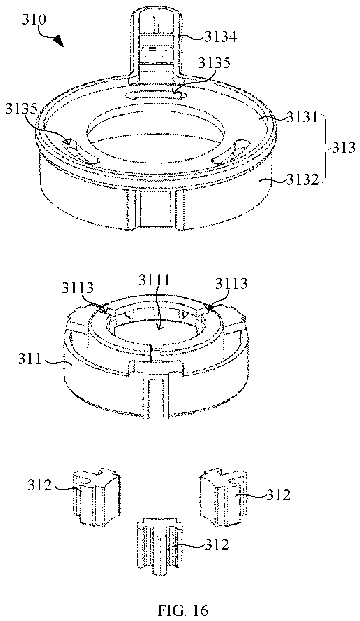

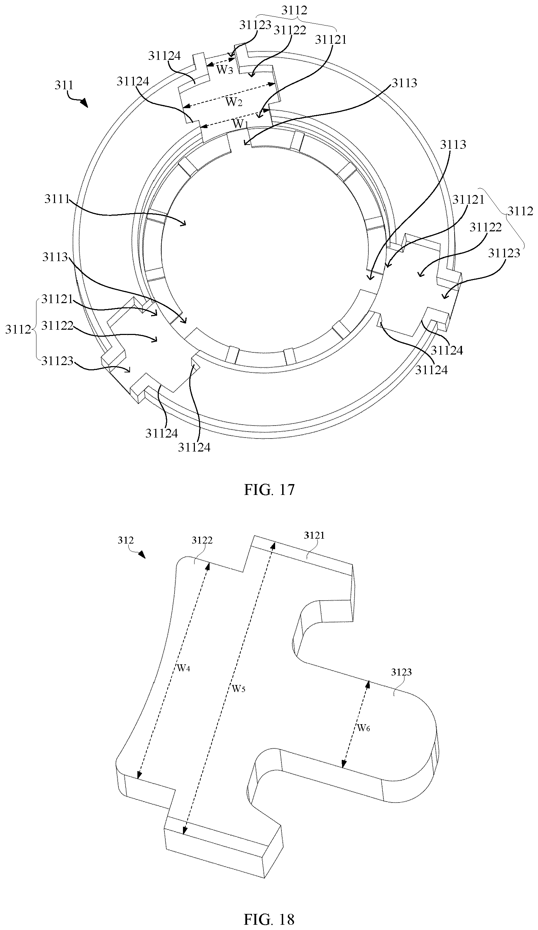

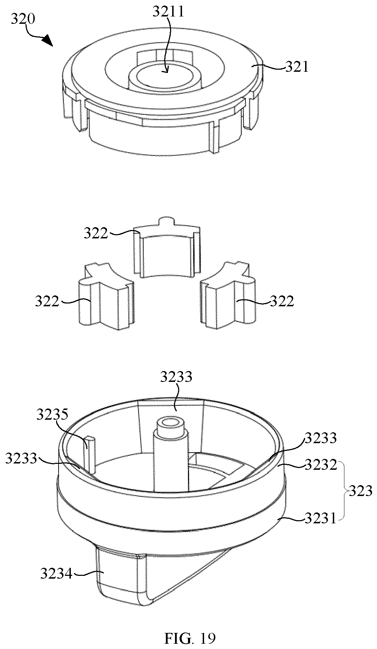

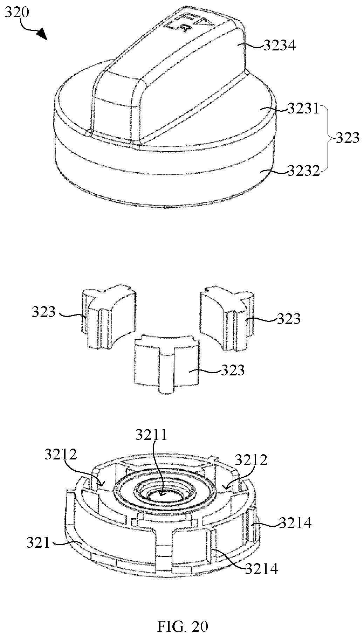

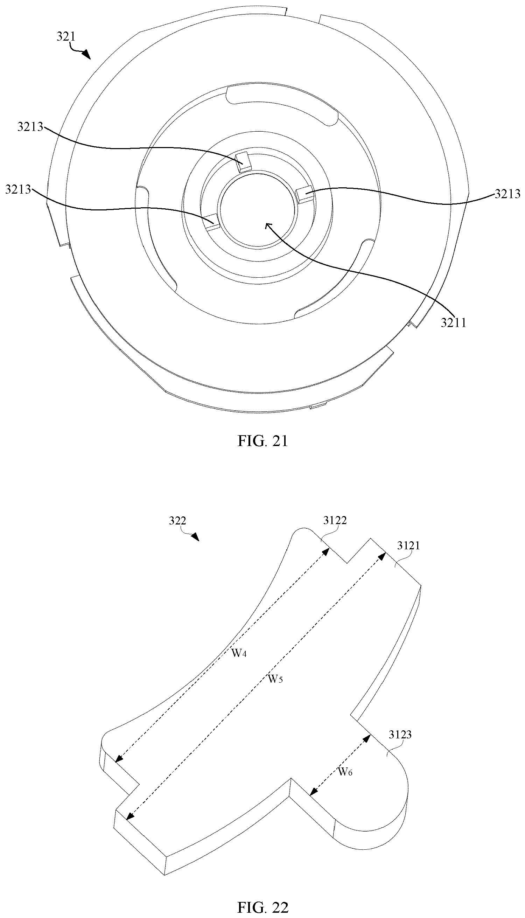

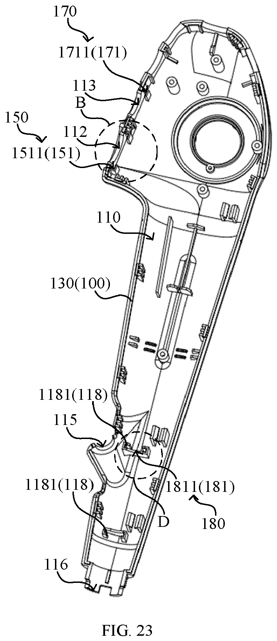

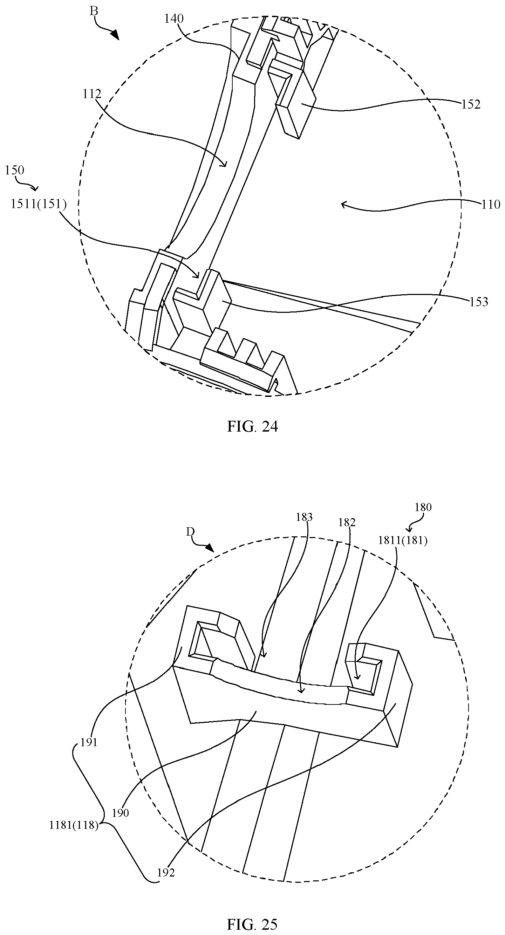

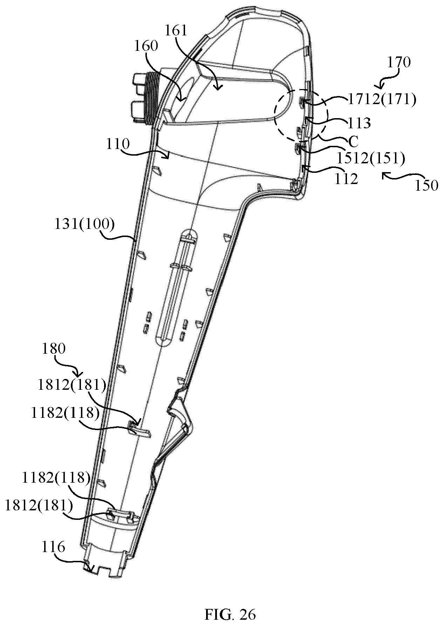

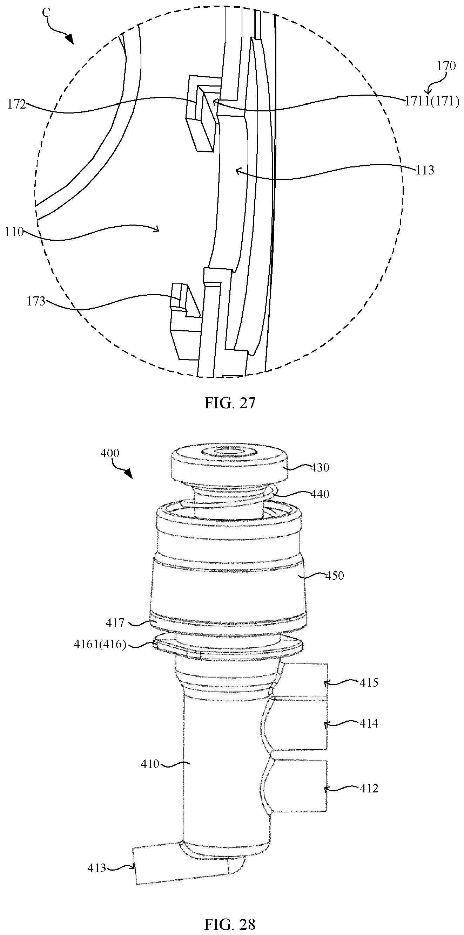

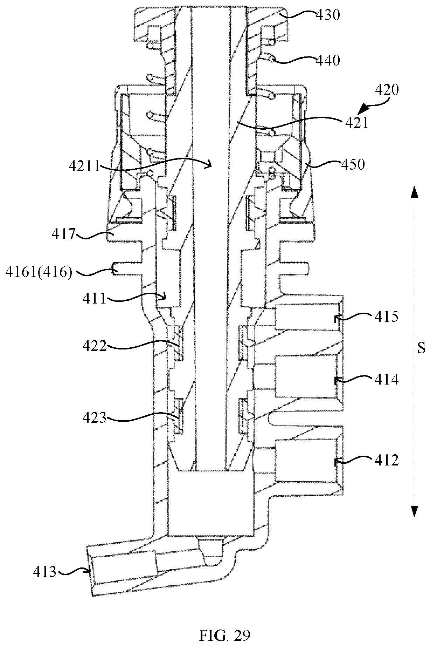

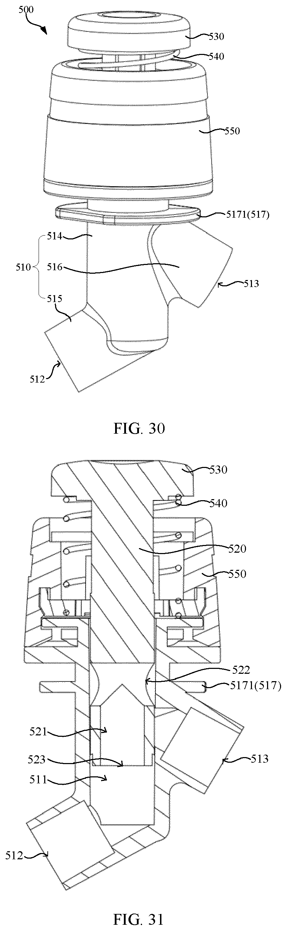

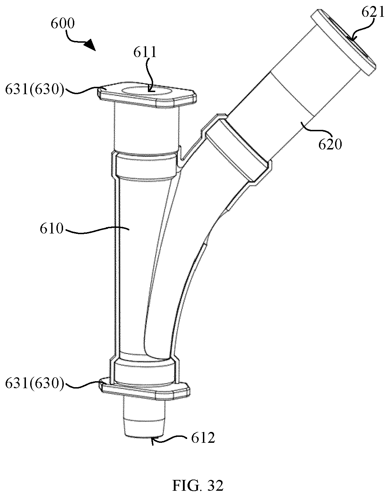

FIG. 1 is a schematic structural view of an endoscope according to an embodiment; FIG. 2 is a first cross-sectional view showing an internal structure of the endoscope according to an embodiment; FIG. 3 is a second cross-sectional view of a handle of the endoscope according to an embodiment; FIG. 4 is an enlarged schematic view of a portion A shown in FIG. 3; FIG. 5 is an exploded schematic view of the endoscope according to an embodiment; FIG. 6 is a schematic structural view of a first pulley according to an embodiment; FIG. 7 is a first schematic view of a first knob according to an embodiment; FIG. 8 is a second schematic view of the first knob according to an embodiment; FIG. 9 is a first schematic view of a second bracket according to an embodiment; FIG. 10 is a second schematic view of the second bracket according to an embodiment; FIG. 11 is a first schematic view of a second pulley according to an embodiment; FIG. 12 is a second schematic view of the second pulley according to an embodiment; FIG. 13 is a first schematic view of a second knob according to an embodiment; FIG. 14 is a second schematic view of the second knob according to an embodiment; FIG. 15 is an exploded view of a first braking assembly according to an embodiment from one perspective; FIG. 16 is an exploded view of the first braking assembly according to an embodiment from another perspective; FIG. 17 is a schematic structural view of a first fixed frame according to an embodiment; FIG. 18 is a schematic structural view of a first braking member according to an embodiment; FIG. 19 is an exploded view of a second braking assembly according to an embodiment from one perspective; FIG. 20 is an exploded view of the second braking assembly according to an embodiment from another perspective; FIG. 21 is a schematic structural view of a second fixed frame according to an embodiment; FIG. 22 is a schematic structural view of a second braking member according to an embodiment; FIG. 23 is a schematic structural view of a first housing according to an embodiment; FIG. 24 is an enlarged schematic view of a portion B shown in FIG. 23; FIG. 25 is an enlarged schematic view of a portion D shown in FIG. 23; FIG. 26 is a schematic structural view of a second housing according to an embodiment.; FIG. 27 is an enlarged schematic view of a portion C shown in FIG. 26; FIG. 28 is a schematic structural view of a gas-water valve according to an embodiment; FIG. 29 is a cross-sectional view of the gas-water valve according to an embodiment; FIG. 30 is a schematic structural view of a negative pressure valve according to an embodiment; FIG. 31 is a cross-sectional view of the negative pressure valve according to an embodiment; FIG. 32 is a schematic structural view of a three-way valve according to an embodiment.

100, Housing; 110, Accommodating Cavity; 111, First Mounting Hole; 112, Second Mounting Hole; 113, Third Mounting Hole; 114, Tube Bracket 115, First Opening; 116, Second Opening; 118, Protruding Base; 1181, First Protruding Base; 1182, Second Protruding Base; 120, Limiting Post; 121, Second Positioning Part; 130, First Housing; 131, Second Housing; 140, Step Part; 150, First Coupling Part; 151, First Slot; 1511, First Type Of Slot; 1512, Second Type Of Slot; 152, First Enclosure Plate; 153, Second Enclosure Plate; 160, Introducing Hole; 161, First Groove; 170, Third Coupling Part; 171, Second Slot; 1711, Third Type Of Slot; 1712, Fourth Type Of Slot; 172, Third Enclosure Plate; 173, Fourth Enclosure Plate; 180, Fifth Coupling Part; 181, Third Slot; 1811, Fifth Type Of Slot; 1812, Sixth Type Of Slot; 182, Second Groove; 183, Notch; 190, Support Plate; 191, First Side Plate; 192, Second Side Plate; 200, Knob Device; 210, First Pulley; 211, First Through Hole; 212, First Fitting Part; 2121, First Fitting Groove; 213, First Buckle; 2131, First Engaging Block; 2132, The First Alignment Part; 214, First Winding Part; 215, First Mounting Part; 216, Third Limiting Part; 217, Second Sealing Ring; 220, Second Pulley; 221, Second Through Hole; 222, Second Fitting Part; 2221, Second Fitting Groove; 223, Second Buckle; 2231, Second Engaging Groove; 2232, Third Alignment Part; 224, Second Winding Part; 225, Second Mounting Part; 226, Fourth Limiting Part; 230, First Knob; 231, Third Fitting Part; 2311, First Fitting Block; 232, Third Buckle; 2321, First Engaging Groove; 2322, Second Alignment Part; 233, First Protrusion; 234, First Through Hole; 240, Second Knob; 241, Fourth Fitting Part; 2411, Second Fitting Block; 242, Fourth Buckle; 2421, Second Engaging Block; 2422, Fourth Alignment Part; 243, Installation Groove; 244, Second Protrusion; 245, Braking Ring; 246, Second Through Hole; 250, Second Bracket; 251, First Limiting Part; 252, Second Limiting Part; 253, First Sealing Ring; 260, First Bracket; 261, Fourth Positioning Part; 262, First Fixing Hole; 300, Braking Device; 310, First Braking Assembly; 311, First Fixed Frame; 3111, First Working Chamber; 3112, First Sliding Groove; 31121, First Groove Section; 31122, Second Groove Section; 31123, Third Groove Section; 31124, Limiting Wall; 3113, First Positioning Part; 312, First Braking Member; 3121, Main Body; 3122, Holding Part; 3123, Transmission Part; 313, First Braking Button; 3131, First Button Body; 3132, First Ring; 3133, First Pushing Part; 3134, First Toggle Part; 3135, Arc-Shaped Groove; 320, Second Braking Assembly; 321, Second Fixed Frame; 3211, Second Working Chamber; 3212, Second Sliding Groove; 3213, Third Positioning Part; 3214, Second Limiting Block; 322, Second Braking Member; 323, Second Braking Button; 3231, Second Button Body; 3232, Second Ring; 3233, Second Pushing Part; 3234, Second Toggle Part; 3235, First Limiting Block; 3236, Second Fixing Hole; 400, Gas-Water Valve; 410, First Valve Body; 411, First Flow Channel; 412, Gas Inlet; 413, Gas Outlet; 414, Liquid Inlet; 415, Liquid Outlet; 416, Second Coupling Part; 4161, First Insertion Block; 417, Protruding Part; 420, First Valve Core; 421, First Valve Stem; 4211, Channel; 422, First Isolation Member; 423, Second Isolation Member; 430, First Valve Cover; 440, First Elastic Member; 450, First Valve Sleeve; 500, Negative Pressure Valve; 510, Second Valve Body; 511, Second Flow Channel; 512, Inlet; 513, Outlet; 514, Body Section; 515, First Branch Section; 516, Second Branch Section; 517, Fourth Coupling Part; 5171, Second Insertion Block; 520, Second Valve Core; 521, Third Flow Channel; 522, Second Aperture; 523, First Aperture; 530, Second Valve Cover; 540, Second Elastic Member; 550, Second Valve Sleeve; 600, Three-Way Valve; 610, First Tube; 611, First Port; 612, Third Port; 620, Second Tube; 621, Second Port; 630, Sixth Coupling Part; 631, Third Insertion Block; 700, Fastener; 800, Bending Section; 900, Distal Tip; 1000, Device Channel Valve.

### DETAILED DESCRIPTION

In order to make the above objects, features and advantages of the present application more obvious and easier to understand, the specific embodiments of the present application will be described in detail below with reference to the accompanying drawings. In the following description, numerous specific details are set forth in order to provide a thorough understanding of the present application. However, the present application can be implemented in many other ways different from those described herein. Those skilled in the art can make similar improvements without departing from the connotation of the present application. Therefore, the present application is not limited to the specific embodiments disclosed below.

In an embodiment, referring to FIGS. 1, 2, 3 and 4, an endoscope includes: a housing 100, a knob device 200, a braking device 300, and a bending section 800. The housing 100 is provided with a first mounting hole 111 that is in communication with the inside and outside of the housing 100. The knob device 200 includes: a first pulley 210 and a second pulley 220 that are rotatably mounted in the housing 100; and a first knob 230 and a second knob 240 that are located outside the housing 100. The first pulley 210 extends out of the housing 100 through the first mounting hole 111. A first through hole 211 extends through the first pulley 210. The second pulley 220 extends out of the housing 100 through the first through hole 211. The first knob 230 is sleeved on the first pulley 210. The second knob 240 is stacked on the first knob 230 and sleeved on the second pulley 220. The braking device 300 includes a first braking assembly 310 and a second braking assembly 320. The first braking assembly 310 is disposed between the first pulley 210 and the housing 100, and is configured to brake the first knob 230 or the first pulley 210. The second braking assembly 320 is disposed on the side of the second knob 240 facing away from the first knob 230, and configured to brake the second knob 240 or the second pulley 220. The bending section 800 is located outside the housing 100, and is respectively connected to the first pulley 210 and the second pulley 220 through a pulling wire.

When the above-mentioned endoscope is in use, the first knob 230 is rotated to drive the first pulley 210 to rotate in the housing 100; after the first pulley 210 is rotated, one side of the bending section 800 is pulled by the pulling wire to enable the bending section 800 to bend in an up-down direction. In order to achieve a fixation at a specific angle, the first pulley 210 or the first knob 230 is braked by the first braking assembly 310 so that the pulling wire cannot be pulled to move, and thus the bending section 800 remains bent at a specific angle. Similarly, the second knob 240 is rotated to drive the second pulley 220 to rotate in the housing 100; after the second pulley 220 is rotated, one side of the bending section 800 is pulled by the pulling wire to enable the bending section 800 to bend in a left-right direction. In order to achieve a fixation at a specific angle, the second pulley 220 or the second knob 240 is braked by the second braking assembly 320 so that the pulling wire cannot be pulled to move, and thus the bending section 800 remains bent at a specific angle. In this way, through the first knob 230, the second knob 240, the first braking assembly 310, and the second braking assembly 320, the control of the bending of the bending section 800 in four directions can be stably realized, thereby improving the stability of the operation. In addition, in terms of structural layout, the second pulley 220 extends through the first pulley 210 through the first through hole 211. In addition, the first knob 230 and the second knob 240 are stacked on the outside of the housing 100, and the first braking assembly 310 is located between the first pulley 210 and the housing 100, and the second braking assembly 320 is disposed on the side of the second knob 240 facing away from the first knob 230. Through such arrangement, the endoscope has more compact structure, and more reasonable arrangement. As such, during operation, it is convenient for the operator to accurately and quickly distinguish the bending in the up-down direction and the bending in the left-right direction of the bending section 800, which makes the operation more convenient, and is beneficial to the reliability of the operation.

Further, referring to FIGS. 5 and 15, the first braking assembly 310 includes: a first fixed frame 311; a first braking button 313; and at least two first braking members 312 slidably disposed on the first fixed frame 311. The first fixed frame 311 is positioned on the housing 100, and the first braking button 313 is sleeved outside the first fixed frame 311. When the first braking button 313 is rotated, the first braking button 313 can push the first braking member 312 to slide to hold the first knob 230 or the first pulley 210 tightly. It can be seen from this that when braking the first pulley 210, the first braking button 313 is rotated in a preset angle to drive the at least two first braking members 312 to slide, so that the at least two first braking members 312 are tightly held on the first knob 230 or the first pulley 210, and thus the first pulley 210 cannot rotate to continue bending the bending section 800.

It should be noted that the "preset angle" should be construed as that: when the first pulley 210 needs to be braked, after the first braking button 313 rotates a certain angle clockwise or counterclockwise around its rotation axis, the first braking button 313 pushes the first braking member 312 to move. Such angle can take any angle value in a range from 0° (excluding 0°) to 360° (excluding 360°).

Furthermore, referring to FIGS. 4 and 15, the first fixed frame 311 is provided with a first working chamber 3111. The first knob 230 extends into the first working chamber 3111 and is sleeved on the first pulley 210. When the first braking button 313 rotates, the first braking button 313 can push the first braking member 312 to extend into the first working chamber 3111 and hold the first knob 230 tightly. In this way, the first knob 230 is connected to the first pulley 210 through the first working chamber 3111.

In an embodiment, referring to FIGS. 4 and 15, at least two first sliding grooves 3112 are spaced apart on the first fixed frame 311 and arranged around the first working chamber 3111. An end of each of the first sliding grooves 3112 is in communication with the first working chamber 3111, and another end of each of the first sliding grooves 3112 is in communication with the outside of the first fixed frame 311. The first braking members 312 are slidably mounted in the first sliding grooves 3112 in a one-to-one correspondence. The first braking member 312 extends through the first sliding groove 3112 and extends out of the first fixed frame 311. At least two first pushing parts 3133 are spaced apart on the inner wall of the first braking button 313. When the first braking button 313 rotates in the preset angle, each first pushing part 3133 pushes the respective one of the first braking members 312 to move toward the first working chamber 3111 to hold the first knob 230 tightly.

It can be seen from the above that the first braking members 312 are slidably disposed in the first sliding grooves 3112 in a one-to-one correspondence, and it is ensured that the first braking member 312 extends through the first sliding groove 3112 and extends out of the first fixed frame 311. When the first braking button 313 sleeved outside the first fixed frame 311 rotates in the preset angle, the first pushing part 3133 on the inner wall of the first braking button 313 contacts the first braking member 312 and pushes the first braking member 312 to move in the first sliding groove 3112. Since the first sliding groove 3112 is in communication with the first working chamber 3111, the first pushing parts 3133 pushes the first braking members 312 respectively to extend into the first working chamber 3111, and is finally held tightly on the first knob 230 to realize the braking of the first pulley 210. In this way, through the cooperating structure between the first braking member 312 and the first braking button 313, the first braking member 312 is directly driven to move in the first sliding groove 3112 during the braking process, so that the first pulley 210 can be braked. The cooperating structure is ingenious in design, and simple, and the braking force is transmitted directly and effectively, ensuring a better braking effect of the knob.

Further, referring to FIGS. 4 and 15, the first braking button 313 includes a first button body 3131 and a first ring 3132 disposed on the first button body 3131. The first ring 3132 is sleeved outside the first fixed frame 311. The first pushing part 3133 is disposed on the inner wall of the first ring 3132. In this way, when the first ring 3132 is driven to rotate outside the first fixed frame 311, the first pushing part 3133 on the inner wall of the first ring 3132 contacts the extended portion of the first braking member 312 and pushes the first braking member 312 to move.

Optionally, the first pushing part 3133 may be connected onto the first ring 3132 by means of, but not limited to, bolt connection, threaded sleeved connection, engaging, welding, riveting, pin connection, integrally molding, etc.

Further, referring to FIGS. 4 and 15, the first pushing part 3133 is formed by the inner wall of the first ring 3132 protruding toward the center of the first ring 3132, that is, the first pushing part 3133 and the first ring 3132 are integrally formed, which makes its production more convenient.

Specifically, referring to FIG. 15, three first pushing parts 3133, three first braking members 312, and three first sliding grooves 3112 are provided. The three first sliding grooves 3112 are arranged at intervals in the circumferential direction of the first working chamber 3111. The three first pushing parts 3133 are arranged at intervals in the circumferential direction of the first ring 3132.

In an embodiment, referring to FIG. 15, the first braking button 313 is provided with a first toggle part 3134. The first toggle part 3134 protrudes and extends in the radial direction of the first braking button 313, to facilitate the operator to rotate the first braking button 313 to improve the operability of the product.

In an embodiment, referring to FIGS. 5 and 16, the first fixed frame 311 is provided with a first positioning part 3113. The first positioning part 3113 positionally cooperates with a second positioning part 121 on the housing 100, to limit the first fixed frame 311 from rotating with the first knob 230. In this way, through the cooperation of the first positioning part 3113 and the second positioning part 121, the first fixed frame 311 is in a relatively fixed state, so that the first braking member 312 holds the first knob 230 tightly.

Optionally, the first positioning part 3113 has a convex structure, and the second positioning part 121 has a hole-like or groove-like structure. Alternatively, the first positioning part 3113 has a hole or groove-like structure, and the second positioning part 121 has a convex structure.

Specifically, referring to FIGS. 5 and 16, the first positioning part 3113 is a positioning groove on the first fixed frame 311. The second positioning part 121 is a positioning protrusion.

Further, referring to FIG. 17, a plurality of first positioning parts 3113 are provided. The plurality of first positioning parts 3113 are arranged at intervals around the first working chamber 3111. In this way, the plurality of first positioning parts 3113 cooperate with the plurality of second positioning parts 121, so that the limiting effect of the rotation of the first fixed frame 311 is better.

In an embodiment, referring to FIGS. 5 and 16, the first braking button 313 is provided with an arc-shaped groove 3135. The arc-shaped groove 3135 is disposed around the first working chamber 3111. The arc-shaped groove 3135 is configured to cooperate with a limiting post 120 on the housing 100 to limit the rotation range of the first braking button 313. In this way, through the first arc-shaped groove 3135, the rotation range of the first braking button 313 can be effectively limited to avoid structural instability caused by excessive rotation.

In an embodiment, referring to FIGS. 4, 6 and 11, the knob device 200 further includes a first bracket 260 disposed in the housing 100. A second through hole 221 extends through the second pulley 220. The first bracket 260 extends through the second through hole 221 and extends out of the housing 100. The second braking assembly 320 is mounted on the first bracket 260. In this way, through the first bracket 260, the second braking assembly 320 can stably brake the second knob 240 or the second pulley 220.

Further, referring to FIGS. 5 and 19, the second braking assembly 320 includes: a second fixed frame 321; a second braking button 323; and at least two second braking members 322 slidably disposed on the second fixed frame 321. The second fixed frame 321 is positioned on the first bracket 260. The second braking button 323 is sleeved outside the second fixed frame 321. When the second braking button 323 rotates, the second braking button 323 can push the second braking member 322 to slide to hold the second knob 240 or the second pulley 220 tightly. It can be seen from this that when braking the second pulley 220, rotating the second braking button 323 in a preset angle drives the at least two second braking members 322 to slide, so that the at least two second braking members 322 are held on the second knob 240 or the second pulley 220 tightly, and thus the second pulley 220 cannot rotate to continue bending the bending section 800.

It should be noted that the "preset angle" should be construed as that: when the second pulley 220 needs to be braked, after the second braking button 323 rotates a certain angle clockwise or counterclockwise around its rotation axis, the second braking button 323 pushes the second braking member 322 to move. Such angle can take any angle value in a range from 0° (excluding 0°) to 360° (excluding 360°).

Further, referring to FIG. 4, the first bracket 260 is provided with a first fixing hole 262 extending therethrough. The second braking button 323 is provided with a second fixing hole 3236 corresponding to the first fixing hole 262. A fastener 700 is inserted into the first fixing hole 262 and the second fixing hole 3236, so that the second braking button 323 is rotatably mounted on the first bracket 260. In this way, through the fastener 700 and the first bracket 260, it can not only ensure that the second braking button 323 is stably mounted on the first bracket 260, but also ensure that the second braking button 323 can rotate relative to the second fixed frame 321, so as to brake the second pulley 220.

It should be noted that when the fastener 700 fixes the second braking button 323, it is not necessary to lock the first bracket 260 and the second braking button 323. On the premise of ensuring that the second braking button 323 cannot be falling off the first bracket 260, it is also necessary to ensure that there is a certain gap between the second braking button 323 and the first bracket 260, so that the second braking button 323 can rotate.

In an embodiment, referring to FIGS. 4 and 5, the second knob 240 is provided with a braking ring 245. The second fixed frame 321 is provided with a second working chamber 3211 into which the braking ring 245 extends. When the second braking button 323 rotates, the second braking button 323 can push the second braking member 322 to extend into the second working chamber 3211 and hold the brake ring 245 tightly. In this way, by disposing the braking ring 245 on the second knob 240, the second braking member 322 can stably hold the second knob 240 tightly, to achieve stable braking.

Further, referring to FIGS. 4 and 13, the side of the second knob 240 facing away from the first knob 230 is provided with an installation groove 243 in which the second braking button 323 is mounted. A first protrusion 233 is provided on the wall of the installation groove 243. The first protrusion 233 is disposed around the second pulley 220. The braking ring 245 is sleeved on the first protrusion 233. In this way, the braking ring 245 is stably mounted, which facilitates the second braking assembly 320 to stably brake the rotation of the second knob 240 and ensure the bending angle of the bending section 800 being fixed, so that the operator can observe an image at a specific angle.

In an embodiment, referring to FIGS. 5 and 21, the second fixed frame 321 is provided with a third positioning part 3213. The third positioning part 3213 positionally cooperates with a fourth positioning part 261 on the first bracket 260, to limit the second fixed frame 321 from rotating with the second knob 240. In this way, through the cooperation of the third positioning part 3213 and the fourth positioning part 261, the second fixed frame 321 is in a relatively fixed state, so that the second braking member 322 can hold the second knob 240 tightly.

Optionally, the third positioning part 3213 has a convex structure, and the fourth positioning part 261 has a hole or groove-like structure. Alternatively, the third positioning part 3213 has a hole or groove-like structure, and the fourth positioning part 261 has a convex structure.

Specifically, referring to FIGS. 5 and 21, the third positioning part 3213 is a positioning protrusion on the second fixed frame 321. The fourth positioning part 261 is a positioning groove.

Further, referring to FIG. 21, a plurality of third positioning parts 3213 are provided. The plurality of third positioning parts 3213 are arranged at intervals around the second working chamber 3211. In this way, the plurality of third positioning parts 3213 cooperate with the plurality of fourth positioning parts 261, so that the restriction effect of the rotation of the second fixed frame 321 is better.

In an embodiment, referring to FIGS. 19 and 20, at least two second sliding grooves 3212 are spaced apart on the second fixed frame 321 and arranged around the second working chamber 3211. An end of each of the second sliding grooves 3212 is in communication with the second working chamber 3211, and another end of each of the second sliding grooves 3212 is configured to be in communication with the outside of the second fixed frame 321. The second braking members 322 are slidably mounted in the second sliding grooves 3212 in a one-to-one correspondence. The second braking member 322 extends through the second sliding groove 3212 and extends out of the second fixed frame 321. At least two second pushing parts 3233 are spaced apart on the inner wall of the second braking button 323. When the second braking button 323 rotates in the preset angle, each second pushing part 3233 pushes the respective one of the second braking members 322 to move toward the second working chamber 3211 to hold the second knob 240 tightly.

It can be seen from the above that the second braking members 322 are slidably disposed in the second sliding grooves 3212 in a one-to-one correspondence, and it is ensured that the second braking member 322 extends through the second sliding groove 3212 and extends out of the second fixed frame 321. When the second braking button 323 sleeved outside the second fixed frame 321 rotates in the preset angle, the second pushing part 3233 on the inner wall of the second braking button 323 contacts the second braking member 322 and pushes the second braking member 322 to move in the second sliding groove 3212. Since the second sliding groove 3212 is in communication with the second working chamber 3211, the second braking members 322 respectively extend into the second working chamber 3211 under the push of the second pushing part 3233, and is finally held on the second knob 240 tightly to realize the braking of the second pulley 220. In this way, through the cooperating structure between the second braking member 322 and the second braking button 323, the second braking member 322 is directly driven to move in the second sliding groove 3212 during the braking process, so that the second pulley 220 can be braked. The cooperating structure is ingenious in design, and simple, and the braking force is transmitted directly and effectively, ensuring a better braking effect of the knob.

Further, referring to FIGS. 4 and 19, the second braking button 323 includes a second button body 3231 and a second ring 3232 disposed on the second button body 3231. The second ring 3232 is sleeved outside the second fixed frame 321. The second pushing part 3233 is disposed on the inner wall of the second ring 3232. In this way, when the second ring 3232 is driven to rotate outside the second fixed frame 321, the second pushing part 3233 on the inner wall of the second ring 3232 contacts the extended portion of the second braking member 322 and pushes the second braking member 322 to move.

Optionally, the second pushing part 3233 may be connected onto the second ring 3232 by means of, but not limited to, bolt connection, threaded sleeve connection, engaging, welding, riveting, pin connection, integrally molding, etc.

Further, referring to FIG. 19, the second pushing part 3233 is formed by the inner wall of the second ring 3232 protruding toward the center of the second ring 3232, that is, the second pushing part 3233 and the second ring 3232 are integrally formed, which makes its production more convenient.

Specifically, three second pushing parts 3233, three second braking members 322 and three second sliding grooves 3212 are provided. The three second sliding grooves 3212 are arranged at intervals in the circumferential direction of the second working chamber 3211. The three second pushing parts 3233 are arranged at intervals in the circumferential direction of the second ring 3232.

In an embodiment, referring to FIG. 20, the second braking button 323 is provided with a second toggle part 3234. The second toggle part 3234 protrudes and extends in the radial direction of the second braking button 323 to facilitate the operator to rotate the second braking button 323 to improve the operability of the product.

In an embodiment, referring to FIGS. 19 and 20, a first limiting block 3235 is disposed on the inner wall of the second braking button 323, and a second limiting block 3214 is disposed on the outer wall of the second fixed frame 321. The first limiting block 3235 cooperates with the second limiting block 3214 to limit the rotation range of the second braking button 323.

In an embodiment, referring to FIG. 17, the first sliding groove 3112 and the second sliding groove 3212 each include a first groove section 31121, a second groove section 31122 and a third groove section 31123 that are connected in sequence. The first groove section 31121 is in communication with the first working chamber 3111 or the second working chamber 3211. The third groove section 31123 is configured to be in communication with the outside of the first fixed frame 311 or the second fixed frame 321. The first braking member 312 or the second braking member 322 is slidably mounted in the second groove section 31122. An end of the first braking member 312 or the second braking member 322 extends into the first groove section 31121, and another end of the first braking member 312 or the second braking member 322 extends through the third groove section 31123 and extends out of the corresponding fixed frame. In this embodiment, the first sliding groove 3112 and the second sliding groove 3212 are designed into a three-section structure. Before braking, one end of the first braking member 312 or the second braking member 322 extends through the third groove section 31123 and extends out of the corresponding fixed frame. During braking, the first braking member 312 or the second braking member 322 is pushed so that another end of the first braking member 312 or the second braking member 322 extends into the first working chamber 3111 or the second working chamber 3211 to hold the first knob 230 or the second knob 240 tightly.

Further, referring to FIGS. 18 and 22, the first braking member 312 and the second braking member 322 each include: a main body 3121; and a holding part 3122 and a transmission part 3123 respectively disposed at opposite ends of the main body 3121. The holding part 3122 is located in the first groove section 31121. The transmission part 3123 is located in the third groove section 31123 and partially extends out of the first fixed frame 311 or the second fixed frame 321. Limiting walls 31124 are formed between the first groove section 31121 and the second groove section 31122, and between the second groove section 31122 and the third groove section 31123 respectively. The main body 3121 is slidably mounted in the second groove section 31122, and is in a limiting fit with the limiting wall 31124 between the first groove section 31121 and the second groove section 31122, and between the second groove section 31122 and the third groove section 31123 respectively. During the limiting fit, when the main body 3121 is fitted with the limiting wall 31124 adjacent to the third groove section 31123, the transmission part 3123 partially extends through the third groove section 31123 and extends out of the fixed frame; when the main body 3121 is fitted with the limiting wall 31124 adjacent to the first groove section 31121, the holding part 3122 extends through the first groove section 31121 and extends into the working chamber. In this way, the limiting walls 31124 between the first groove section 31121 and the second groove section 31122, and between the second groove section 31122 and the third groove section 31123 are used to limit the movement stroke of the main body 3121 in the second groove section 31122, so that on the premise of achieving effective braking, it is possible to avoid the first braking member 312 or the second braking member 322 from slipping out and causing the braking device 300 to fail.

Optionally, the holding part 3122 and the transmission part 3123 may be connected onto the main body 3121 by means of, but not limited to, bolt connection, threaded sleeve connection, engaging connection, welding, riveting, pin connection, integrally molding, etc. The integrally molding should be construed as: injection molding, extrusion molding, die-casting, etc.

Specifically, referring to FIGS. 18 and 22, the transmission part 3123, the main body 3121 and the holding part 3122 are integrally formed, which not only helps to simplify the manufacturing process, but also helps to ensure that the first braking member 312 or the second braking member 322 is stable.

Certainly, when the first braking member 312 and the second braking member 322 are each integrally formed, there are many choices for their shape design, such as an "E" shape rotated by 90° counterclockwise, and in this regard, which is not specifically limited in this embodiment.

In an embodiment, referring to FIG. 17, the width W₁ of the first groove section 31121 and the width W₃ of the third groove section 31123 are both less than the width W₂ of the second groove section 31122, so that the limiting walls 31124 are formed between the first groove section 31121 and the third groove section 31123, and between the first groove section 31121 and the second groove section 31122 respectively. The width W₄ of the holding part 3122 and the width W₆ of the transmission part 3123 are both less than the width W₅ of the main body 3121. It can be seen from this that during the braking process, the width difference between the second groove section 31122 and the first groove section 31121 and between the second groove section 31122 and the third groove section 31123 is utilized to form the limiting walls 31124 on both sides, to stably limit the main body 3121 to move back and forth in the second groove section 31122 to ensure that the braking structure is more stable.

It should be noted that the first groove section 31121 is located in the middle of the second groove section 31122. The width difference between the first groove section 31121 and the second groove section 31122 can be utilized to form two limiting walls 31124. Certainly, when the first groove section 31121 is not located in the middle of the second groove section 31122, one limiting wall 31124 can also be formed. In the same way, the third groove section 31123 is located in the middle of the second groove section 31122. The width difference between the third groove section 31123 and the second groove section 31122 can be utilized to form two limiting walls 31124. Certainly, when the third groove section 31123 is not disposed in the middle of the second groove section 31122, and one limiting wall 31124 can also be formed.

In an embodiment, referring to FIGS. 4, 6 and 7, the first pulley 210 is provided with a first fitting part 212 and a first buckle 213. The first fitting part 212 and the first buckle 213 are both located outside the housing 100. The second pulley 220 is provided with a second fitting part 222 and a second buckle 223. The second fitting part 222 and the second buckle 223 are both located outside the housing 100. The first knob 230 is provided with a third fitting part 231 and a third buckle 232 buckled to the first buckle 213. The third fitting part 231 cooperates with the first fitting part 212 so that the first knob 230 can drive the first pulley 210 to rotate. The second knob 240 is provided with a fourth fitting part 241 and a fourth buckle 242 buckled to the second buckle 223. The fourth fitting part 241 cooperates with the second fitting part 222 so that the second knob 240 can drive the second pulley 220 to rotate.

During the assembling process, the first pulley 210 is rotatably mounted in the housing 100 so that the first pulley 210 extends out of the housing 100; then, the first knob 230 is sleeved on the portion of the first pulley 210 extending out of the housing 100, and the first fitting part 212 is fitted with the third fitting part 231, and the first buckle 213 is buckled to the third buckle 232, so that the first pulley 210 and the first knob 230 are reliably connected to each other, ensuring the first pulley 210 is stably mounted in the housing 100. In addition, under the action of the first fitting part 212 and the third fitting part 231, the first knob 230 can drive the first pulley 210 to rotate, so as to stably control the bending of the bending section 800. Similarly, the second pulley 220 is rotatably mounted in the housing 100 so that the second pulley 220 extends through the first through hole 211 and extends out of the housing 100; then, the second knob 240 is sleeved on the portion of the second pulley 220 extending out of the housing 100, and the second fitting part 222 is fitted with the fourth fitting part 241, and the second buckle 223 is buckled to the fourth buckle 242, so that the second pulley 220 and the second knob 240 are reliably connected to each other, ensuring that the second pulley 220 is stably mounted in the housing 100. In addition, under the action of the second fitting part 222 and the fourth fitting part 241, the second knob 240 can drive the second pulley 220 to rotate, so as to stably control the bending of the bending section 800. The overall structure is ingeniously designed, and the first knob 230 and the second knob 240 can be mounted separately by just being sleeved, which makes the product assembly convenient, helps improve the assembling efficiency, and effectively reduces the production cost.

It should be noted that the first fitting part 212 and the third fitting part 231 can limit the relative rotation between the first pulley 210 and the first knob 230, so that the first knob 230 can drive the first pulley 210 to rotate together. The structures of the first fitting part 212 and the third fitting part 231 can be variously designed. For example, the first fitting part 212 has a convex structure, and the second fitting part 222 has a groove-like or hole-like structure; alternatively, the first fitting part 212 has a groove-like or hole-like structure, the second fitting part 222 has a convex structure, etc.

Similarly, the second fitting part 222 and the fourth fitting part 241 can limit the relative rotation between the second pulley 220 and the second knob 240, so that the second knob 240 can drive the second pulley 220 to rotate together. The structures of the second fitting part 222 and the fourth fitting part 241 can be variously designed. For example, the second fitting part 222 has a convex structure, and the fourth fitting part 241 has a groove-like or hole-like structure; alternatively, the second fitting part 222 has a groove-like or hole-like structure, the fourth fitting part 241 has a convex structure, etc.

It should also be noted that when the first buckle 213 cooperates with the third buckle position 232, the movement of the first pulley 210 can be limited to prevent the first pulley 210 from falling off the mounting hole of the housing 100 to ensure that the first pulley 210 is stably mounted inside the housing 100. In addition, when the second buckle 223 cooperates with the fourth buckle 242, the movement of the second pulley 220 can be limited, preventing the second pulley 220 from falling off the mounting hole of the housing 100, so as to ensure that the second pulley 220 can be stably mounted in the housing 100.

Further, referring to FIGS. 4 and 9, the knob device 200 further includes a second bracket 250 mounted in the housing 100. The second bracket 250 is sleeved on the second pulley 220 and covers the first pulley 210. In this way, the first wire pulley 210 is stably mounted in the housing 100 through the second bracket 250. In addition, the second bracket 250 can also provide protection for the first pulley 210, so that the pulling wire can be stably wound around the first pulley 210.

Optionally, the second bracket 250 may be connected to the housing 100 by means of, but not limited to, bolt connection, engagement, riveting, welding, bonding, etc.

Further, referring to FIG. 4, the knob device 200 further includes a first sealing ring 253. The first sealing ring 253 is disposed between the second bracket 250 and the second pulley 220, to improve the sealing performance between the second bracket 250 and the second pulley 220 and prevent liquid from infiltrating between the second bracket 250 and the second pulley 220 to corrode and damage the first pulley 210.

Certainly, referring to FIG. 4, the knob device 200 further includes a second sealing ring 217. The second sealing ring 217 is disposed between the first pulley 210 and the housing 100 to further improve the sealing performance of the knob device 200 and effectively prevent liquid from infiltrating from the outside of the housing 100 into the first pulley 210 through the mounting hole.

In an embodiment, referring to FIGS. 6, 9, 10 and 12, the second bracket 250 is provided with a first limiting part 251 and a second limiting part 252 respectively. The first pulley 210 is provided with a third limiting part 216. The third limiting part 216 cooperates with the first limiting part 251 to limit the rotation angle of the first pulley 210. The second pulley 220 is provided with a fourth limiting part 226. The fourth limiting part 226 cooperates with the second limiting part 252 to limit the rotation angle of the second pulley 220. It can be seen from the above that when the first knob 230 drives the first pulley 210 to rotate in the preset angle, the third limiting part 216 cooperates with the first limiting part 251 to limit the first pulley 210 from continuing to rotate, thereby avoiding the pulling wire from being broken due to excessive rotation of the first pulley 210, thereby ensuring the use safety of the endoscope. Similarly, when the second knob 240 drives the second pulley 220 to rotate in the preset angle, the fourth limiting part 226 cooperates with the second limiting part 252 to limit the second pulley 220 from continuing to rotate, thereby preventing the pulling wire from being broken due to excessive rotation of the second pulley 220, thereby ensuring the use safety of the endoscope.

It should be noted that the first limiting part 251, the second limiting part 252, the third limiting part 216 and the fourth limiting part 226 all have a convex structure, and the friction between the convex structures is utilized, so that the rotation ranges of the first pulley 210 and the second pulley 220 are effectively controlled.

Further, referring to FIGS. 6 and 7, at least two first buckles 213 and at least two third buckles 232 are provided. The at least two first buckles 213 are arranged at intervals in the circumferential direction of the first pulley 210. The at least two third buckles 232 and the at least two first buckles 213 are arranged in a one-to-one correspondence. In this way, the first buckles 213 cooperate with the third buckles 232 in a one-to-one correspondence, so that the first pulley 210 and the first knob 230 are effectively buckled. As such, the connection between the first pulley 210 and the first pulley 210 is more reliable and stable.

In an embodiment, referring to FIGS. 6 and 7, the first buckle 213 is a first engaging block 2131. The third buckle 232 is a first engaging groove 2321 adapted to the first engaging block 2131. In this way, the first engaging block 2131 cooperates with the first engaging groove 2321, so that the first pulley 210 and the first knob 230 are stably connected to each other.

In addition, since one of the first engaging blocks 2131 is provided with a first alignment part 2132, and one of the first engaging grooves 2321 is provided with a second alignment part 2322 on the groove wall thereof and the second alignment part 2322 cooperates with the first alignment part 2132, during buckling, it is only necessary to align the first alignment part 2132 with the second alignment part 2322, and the first knob 230 can be stably buckled onto the first pulley 210, making the assembling of the knob device 200 more convenient, thus helping to improve the assembling efficiency of the endoscope.

It should be noted that the first alignment part 2132 and the second alignment part 2322 have various forms. For example, the first alignment part 2132 is designed as a bump and the second alignment part 2322 is designed as a groove, alternatively, the first alignment part 2132 and the second alignment part 2322 are both designed with corresponding patterns, logos, etc.

Specifically, the first alignment part 2132 is a groove, and the second alignment part 2322 is a bump.

Further, referring to FIGS. 11 and 13, at least two second buckles 223 and at least two fourth buckles 242 are provided. The at least two second buckles 223 are arranged at intervals in the circumferential direction of the second pulley 220. The at least two fourth buckles 242 and the at least two second buckles 223 are arranged in a one-to-one correspondence. In this way, the second buckles 223 cooperate with the fourth buckles 242 in a one-to-one correspondence, so that the second pulley 220 and the second knob 240 are effectively buckled. As such, the connection between the second pulley 220 and the second knob 240 is more reliable and stable.

In an embodiment, referring to FIGS. 11 and 13. the second buckle 223 is a second engaging groove 2231, and the fourth buckle 242 is a second engaging block 2421 adapted to the second engaging groove 2231. In this way, the second engaging block 2421 cooperates with the second engaging groove 2231, so that the second pulley 220 and the second knob 240 are stably connected to each other.

In addition, since one of the second engaging grooves 2231 is provided with a third alignment part 2232 on the groove wall thereof, and one of the second engaging blocks 2421 is provided with a fourth alignment part 2422 that cooperates with the third alignment part 2232, during buckling, it is only necessary to align the third alignment part 2232 with the fourth alignment part 2422, and the second knob 240 can be stably buckled onto the second pulley 220, making the assembling of the knob device 200 more convenient., thus helping to improve the assembling efficiency of the endoscope.

It should be noted that the third alignment part 2232 and the fourth alignment part 2422 have various forms. For example, the third alignment part 2232 is designed as a groove and the fourth alignment part 2422 is designed as a bump; alternatively, the third alignment part 2232 and the fourth alignment part 2422 are both designed with corresponding patterns, logos, etc.

Specifically, the third alignment part 2232 is a bump, and the fourth alignment part 2422 is a groove.

Further, referring to FIGS. 6 and 8, at least two first fitting parts 212 and at least two third fitting parts 231 are provided. The at least two first fitting parts 212 are arranged at intervals in the circumferential direction of the first pulley 210. The at least two third fitting parts 231 are provided in a one-to-one correspondence with the at least two first fitting parts 212. In this way, the first fitting parts 212 cooperate with the third fitting parts 231 in a one-to-one correspondence, so that the first pulley 210 and the first knob 230 effectively fitted to each other, so that the first knob 230 drives the first pulley 210 more reliably and stably.

In an embodiment, referring to FIGS. 6 and 8, the first fitting part 212 is a first fitting groove 2121. The third fitting part 231 is a first fitting block 2311 adapted to the first fitting groove 2121. The first fitting groove 2121 extends in the axial direction of the first pulley 210. In this way, the first fitting block 2311 is inserted into the first fitting groove 2121, so that the first pulley 210 cannot rotate relative to the first knob 230, and thus the first knob 230 can drive the first pulley 210 to rotate together.

Specifically, referring to FIGS. 6 and 8, the first fitting groove 2121 extends from an end of the first pulley 210 in the axial direction of the first pulley 210. In this way, the first fitting block 2311 can be inserted into the first fitting groove 2121 more conveniently, thereby making the connection between the first pulley 210 and the first knob 230 more convenient.

Further, referring to FIGS. 11 and 14, at least two second fitting parts 222 and at least two fourth fitting parts 241 are provided. The at least two second fitting parts 222 are arranged at intervals in the circumferential direction of the second pulley 220. The at least two second fitting parts 222 and the at least two fourth fitting parts 241 are provided in a one-to-one correspondence. In this way, the second fitting parts 222 cooperate with the fourth fitting parts 241 in a one-to-one correspondence, so that the second pulley 220 and the second knob 240 are effectively buckled. As such, the second knob 240 drives the second pulley 220 to rotate more reliably and stably.

In an embodiment, referring to FIGS. 11 and 14, the second fitting part 222 is a second fitting groove 2221. The fourth fitting part 241 is a second fitting block 2411 adapted to the second fitting groove 2221. The second fitting groove 2221 extends in the axial direction of the second pulley 220. In this way, the second fitting block 2411 is inserted into the second fitting groove 2221, so that the second pulley 220 cannot rotate relative to the second knob 240. As such, the second knob 240 can drive the second pulley 220 to rotate together.

Specifically, referring to FIGS. 11 and 14, the second fitting groove 2221 extends from an end of the second pulley 220 in the axial direction of the second pulley 220. In this way, the second fitting block 2411 can be inserted into the second fitting groove 2221 more conveniently, thereby making the connection between the second pulley 220 and the second knob 240 more convenient.

In an embodiment, referring to FIGS. 6, 7 and 8, the first knob 230 is provided with a first through hole 234 through which the first pulley 210 extends. At least two third fitting parts 231 are spaced apart and arranged around the first through hole 234. At least two third buckles 232 are spaced apart and arranged around the first through hole 234. When the first pulley 210 extends into the first through hole 234, the first fitting part 212 is fitted on the third fitting part 231, and the first buckle 213 is buckled on the third buckle 232. In this way, during assembling, the first pulley 210 only needs to be inserted into the first through hole 234 to achieve stable engagement between the second pulley 220 and the second knob 240.

In an embodiment, referring to FIGS. 5 and 8, a first protrusion 233 is provided on a side of the first knob 230 facing the housing 100. The first protrusion 233 is disposed around the first through hole 234, and the first protrusion 233 is configured to cooperate with and brake the first braking assembly 310. In this way, the rotation of the first knob 230 is stably braked by the first braking assembly 310, so that the bending angle of the bending section 800 is fixed, thereby facilitating the operator to observe an image at a specific angle.

In an embodiment, referring to FIGS. 4, 13 and 14, the second knob 240 is provided with a second through hole 246 through which the second pulley 220 extends. At least two fourth fitting parts 241 are spaced apart and arranged around the second through hole 246. At least two fourth buckles 242 are spaced apart and arranged around the second through hole 246. When the second pulley 220 extends into the second through hole 246, the second fitting part 222 is fitted on the fourth fitting part 241, and the second buckle 223 is buckled on the fourth buckle position 242. In this way, during assembling, the second pulley 220 only needs to be inserted into the second through hole 246 to achieve stable engagement between the second pulley 220 and the second knob 240.

In an embodiment, referring to FIG. 6, the first pulley 210 includes a first winding part 214 and a first mounting part 215 disposed on the first winding part 214. The first through hole 211 extends through the first winding part 214 and the first mounting part 215. The first winding part 214 is located in the housing 100. The first mounting portion 215 is inserted into the mounting hole. The first fitting part 212 and the first buckle 213 are both disposed on the first mounting part 215. In this way, the first pulley 210 can be mounted in the housing 100 more stably.

In an embodiment, referring to FIGS. 11 and 12, the second wire pulley 220 includes a second winding part 224 and a second mounting part 225 disposed on the second winding part 224. The second winding part 224 is located in the housing 100, the second mounting part 225 is inserted into the first through hole 211, and the second fitting part 222 and the second buckle 223 are both disposed on the second mounting part 225. In this way, the second pulley 220 can be mounted in the housing 100 more stably.

In an embodiment, referring to FIGS. 1, 23 and 28, the endoscope further includes: a gas-water valve 400; and a distal tip 900 disposed on the bending section 800. The gas-water valve 400 is embedded in the housing 100. A first coupling part 150 is disposed on the inner wall of the housing 100. The gas-water valve 400 is provided with a second coupling part 416 that is engaged with the first coupling part 150. It can be seen from this that during mounting the gas-water valve 400, the gas-water valve 400 is embedded in the housing 100, and the second coupling part 416 is engaged onto the first coupling part 150, so that the gas-water valve 400 can be effectively fixed to complete the mounting of the gas-water valve 400 on the housing 100. Since the gas-water valve 400 is fixed on the housing 100 by engaging, there is no need to prepare a large number of bolts or screws and other parts during mounting, which effectively simplifies the mounting operation and improves the assembling efficiency of the endoscope.

It should be noted that, referring to FIG. 29, the gas-water valve 400 includes a first valve body 410 and a first valve core 420. The housing 100 is provided with a second mounting hole 112 and has an accommodating cavity 110 therein that is in communication with the second mounting hole 112. The first valve body 410 extends through the second mounting hole 112 and extends into the accommodating cavity 110. The first valve body 410 is provided with a second coupling part 416 that is engaged with the first coupling part 150. The first valve body 410 is provided with a first flow channel 411 therein. The first valve body 410 is provided with a gas inlet 412, a gas outlet 413, a liquid inlet 414 and a liquid outlet 415 that are all in communication with the first flow channel 411. The first valve core 420 is movably sleeved in the first valve body 410 and extend through the first flow channel 411. By driving the first valve core 420 to provide a communication between the gas inlet 412 and the gas outlet 413 or between the liquid inlet 414 and the liquid outlet 415. After the gas-water valve 400 is mounted, when switching between gas and liquid, it is only necessary to drive the first valve core 420 to operate in the first valve body 410 to selectively provide a communication between the gas inlet 412 and the gas outlet 413 or between the liquid inlet 414 and the liquid outlet 415, enabling the gas-water valve 400 to deliver liquid or gas to the inside of the organ to clean the camera. As such, this greatly facilitates the gas-liquid switching operation and ensures stable procedure.

It should be noted that the structure of the first valve core 420 is not specifically limited in this embodiment, as long as it can selectively control the communication between the gas inlet 412 and the gas outlet 413 or between the liquid inlet 414 and the liquid outlet 415.

Alternatively, the first coupling part 150 may have a convex structure, and the second coupling part 416 may have a groove-like or hole-like structure; alternatively, the first coupling part 150 may have a groove-like or hole-like structure, and the second coupling part 416 may have a convex structure.

Specifically, referring to FIGS. 24 and 28, the first coupling part 150 is the first slot 151. The second coupling part 416 is a first insertion block 4161 that cooperates with the first slot 151. In this way, the first insertion block 4161 cooperates with the first slot 151, making the fixing of the gas-water valve 400 more convenient.

In an embodiment, referring to FIG. 28, the first insertion block 4161 is extended on the first valve body 410 in the circumferential direction of the first valve body 410. In this way, the first insertion block 4161 can be easily inserted into the first slot 151 in different orientations, thereby ensuring that the gas-water valve 400 can be can be mounted more stably and reliably.

It should be noted that the configuration relationship between the quantity of the first slots 151 and the quantity of the first insertion blocks 4161 can be in a one-to-one, two-to-one or many-to-one correspondence, that is, the same first insertion block 4161 is inserted into two or more first slots 151 at the same time.

In an embodiment, referring to FIGS. 23 and 26, the housing 100 includes a first housing 130 and a second housing 131. The first housing 130 covers the second housing 131, and the accommodating cavity 110 is enclosed by the first housing 130 and the second housing 131. The first slot 151 includes a first type of slot 1511 and a second type of slot 1512. The first type of slot 1511 is defined on the first housing 130. The second type of slot 1512 is defined on the second housing 131. An end of the first insertion block 4161 is inserted into the first type of slot 1511, and another end of the first insertion block 4161 is inserted into the second type of slot 1512. It can be seen from this that when the first housing 130 and the second housing 131 are fitted to each other, the opposite ends of the first insertion block 4161 are inserted into the first type of slot 1511 and the second type of slot 1512 respectively. In this way, the gas-water valve 400 is effectively fixed both in the up-down direction.

Optionally, the first housing 130 and the second housing 131 may be fitted to each other by means of, but not limited to, bolt connection, engaging connection, pin connection, etc.

In an embodiment, referring to FIG. 24, a first enclosure plate 152 and a second enclosure plate 153 are spaced apart on the wall of the accommodating cavity 110. The first enclosure plate 152 and the second enclosure plate 153 are respectively located on opposite sides of the second mounting hole 112 and enclose the first slot 151. It can be seen from this that the first slot 151 and the second mounting hole 112 of this embodiment are arranged facing each other. When the first valve body 410 extends into the second mounting hole 112, the first insertion block 4161 on the first valve body 410 can also be inserted into the first slot 151. As such, the gas-water valve 400 can be mounted more conveniently.

Optionally, the first enclosure plate 152 and the second enclosure plate 153 can be mounted on the housing 100 in a manner such as, but not limited to, bolt connection, engaging, welding, riveting, pin connection, bonding, integrally molding, etc. The integrally molding can include injection molding, 3D printing, etc.

In an embodiment, referring to FIGS. 24 and 28, a step part 140 is disposed on the side of the housing 100 facing away from the accommodating cavity 110. The step part 140 is disposed around the second mounting hole 112. The first valve body 410 is provided with a protruding part 417 in the circumferential direction thereof. The protruding part 417 is in abutting fit with the step part 140 to effectively limit the position of the gas-water valve 400 in the second mounting hole 112, so that the gas-water valve 400 can be stably mounted on the housing 100.

Specifically, referring to FIG. 24, the protruding part 417 and the first insertion block 4161 are arranged side by side and spaced apart on the first valve body 410. In this way, during mounting, the protruding part 417 and the first insertion block 4161 act on the outside and inside of the housing 100 respectively to form an internal limiting structure and an external limiting structure, which effectively prevents the gas-water valve 400 from shaking up and down in the second mounting hole 112, so that the gas-water valve 400 is more stable on the housing 100.

In an embodiment, referring to FIG. 29, the first valve core 420 includes a valve stem and a first isolation member 422 and a second isolation member 423 spaced apart on the valve stem. The valve stem is movably sleeved in the first valve body 410, and extends through the first flow channel 411. The first isolation member 422 and the second isolation member 423 are both sealingly fitted with the inner wall of the first flow channel 411. When the valve stem moves to a first position, the first isolation member 422 is located between the liquid inlet 414 and the liquid outlet 415, blocking the communication between the liquid inlet 414 and the liquid outlet 415. In this case, the liquid inlet 414 and the liquid outlet 415 are both located on one side of the second isolation member 423, and the gas inlet 412 and the gas outlet 413 are located on another side of the second isolation member 423. Such configuration has following purposes: firstly, the channel between the gas and the liquid is blocked by the second isolation member 423 to avoid cross-flow of the gas flow and the liquid flow; secondly, the gas inlet 412 and the gas outlet 413 are located on the same side of the second isolation member 423, so that the gas inlet 412 is in communication with the gas outlet 413, and thus the gas can enter the inside of the organ to blow the camera. In addition, when the valve stem moves to a second position, the second isolation member 423 is located between the gas inlet 412 and the gas outlet 413, blocking the communication between the gas inlet 412 and the gas outlet 413. In this case, the liquid inlet 414 and the liquid outlet 415 are both located on one side of the first isolation member 422, and the liquid inlet 414 and the liquid outlet 415 are both located on another side of the first isolation member 422. Such configuration has following purposes: firstly, the channel between the gas and the liquid is blocked by the first isolation member 422 to avoid cross-flow of the gas flow and the liquid flow; secondly, the liquid inlet 414 and the liquid outlet 415 are located on the same side of the first isolation member 422, so that the liquid inlet 414 is in communication with the liquid outlet 415, and thus the liquid can enter the inside of the organ to flush the camera.

It should be noted that the positional relationship between the gas inlet 412 and the gas outlet 413 on the first valve body 410 is not limited thereto. For example, the gas inlet 412 is above the gas outlet 413; alternatively, the gas inlet 412 is below the liquid outlet 415. Similarly, the positional relationship between the liquid inlet 414 and the liquid outlet 415 on the first valve body 410 is not limited thereto. For example, the liquid inlet 414 is above the liquid outlet 415; alternatively, the liquid inlet 414 is below the liquid outlet 415.

Specifically, referring to FIG. 29, the liquid outlet 415, the liquid inlet 414, the gas inlet 412 and the gas outlet 413 are arranged at intervals from top to bottom in the length direction of the first valve body 410. In order to facilitate understanding of the length direction of the first valve body 410, as shown in FIG. 29, the length direction of the first valve body 410 is the direction indicated by any arrow S in FIG. 29.

In an embodiment, referring to FIG. 28, the gas-water valve 400 further includes a first valve cover 430 and a first elastic member 440. The first valve cover 430 is provided on the valve stem. The first elastic member 440 is connected between the first valve cover 430 and the first valve body 410, and is configured to drive the valve stem to return to the first position from the second position, so that the gas-water valve 400 returns to an initial state, prepared for the next operation. In addition, the gas-water valve 400 further includes a first valve sleeve 450. The first valve sleeve 450 is sleeved on the valve stem, and connected to the first valve body 410.

Optionally, the first elastic member 440 may be a spring, elastic rubber, elastic metal sheet, etc.

In an embodiment, referring to FIG. 29, a channel 4211 is disposed in the valve stem. An end of the channel 4211 is connected to the gas outlet 413, and another end of the channel 4211 is configured to be in communication with the outside. In this way, only when an end of channel 4211 is blocked (for example, blocked by fingers), the valve stem can be in a gas-liquid switching state.

In an embodiment, referring to FIGS. 23 and 30, the endoscope further includes a negative pressure valve 500. The negative pressure valve 500 is embedded in the housing 100. A third coupling part 170 is disposed on the inner wall of the housing 100. The negative pressure valve 500 is provided with a fourth coupling part 517 that is engaged with the third coupling part 170. Since the third coupling part 170 is disposed in the housing 100 and the fourth coupling part 517 is disposed on the negative pressure valve 500, the fourth coupling part 517 can be engaged to the third coupling part 170 when fixed, so that the negative pressure valve 500 is positioned on the housing 100 stably and quickly. Compared with conventional screw or bolt fixing methods, the structure for mounting is greatly simplified, so that the negative pressure valve 500 can be mounted faster and easier, which is beneficial to improving the assembling efficiency of the endoscope.

Further, referring to FIG. 31, the negative pressure valve 500 includes a second valve body 510 and a second valve core 520. The housing 100 is provided with a third mounting hole 113, and has the accommodating cavity 110 therein that is in communication with the second mounting hole 112. The second valve body 510 extends through the third mounting hole 113 and extends into the accommodating cavity 110. The second valve body 510 is provided with a second coupling part 416 that is engaged with the first coupling part 150. The second valve body 510 is provided with a second flow channel 511. The second valve body 510 is provided with an inlet 512 and an outlet 513 that are in communication with the second flow channel 511. The second valve core 520 is movably sleeved in the second valve body 510, and extend through the second flow channel 511. The second valve core 520 is provided with a third flow passage 521 configured to be in communication with the inlet 512 and the outlet 513. The second valve core 520 can be driven to provide a communication between the third flow passage 521 and the inlet 512 and the outlet 513, or to block the inlet 512 and the outlet 513.

After the negative pressure valve 500 is mounted, the third flow channel 521 is in communication with the inlet 512 and the outlet 513 by driving the second valve core 520. In this case, a negative pressure device can generate a negative pressure device at the inlet 512, the third flow channel 521 and the outlet 513, so that the liquid and tissue in the organ are discharged through the inlet 512, the third flow channel 521 and the outlet 513 in sequence. When the drawing is completed or no drawing is needed, the second valve core 520 is driven to block the communication between the inlet 512 and the outlet 513, and the connection between the endoscope and the negative pressure device is disconnected. In this way, when the negative pressure valve 500 switches the opening and closing of the channel, it only needs to drive the second valve core 520 to complete the switching operation, which makes the switching operation more convenient, and helps ensure stable procedure.

It should be noted that driving the second valve core 520 to provide a communication between the third flow channel 521 and the inlet 512 and the outlet 513 or blocking the inlet 512 and the outlet 513 should be construed as that: the second valve core 520 is driven to perform corresponding operations in the second flow channel 511, and when the third flow channel 521 is opposite to the inlet 512 and the outlet 513 respectively, the inlet 512 and the outlet 513 are in communication with each other; when the second valve core 520 blocks at least one of the inlet 512 and the outlet 513, the inlet 512 and the outlet 513 cannot be in communication with each other through the third flow channel 521.

Further, referring to FIG. 26, the housing 100 is provided with an introducing hole 160 that is in communication with the accommodating cavity 110. A connecting tube of the negative pressure device in communication with the outlet 513 extends through the introducing hole 160. When the negative pressure valve 500 is connected to the negative pressure device, the connecting tube of the negative pressure device can extend into the accommodating cavity 110 through the introducing hole 160, and be connected to the negative pressure valve 500 to realize the reliable connection between the negative pressure valve 500 and the negative pressure device, thus greatly facilitating the connection between the negative pressure valve 500 and the negative pressure device.

Further, referring to FIG. 26, the wall of the accommodating cavity 110 is provided with a first groove 161. The first groove 161 is located between the third mounting hole 113 and the introducing hole 160. The first groove 161 is configured to receive the connecting tube of the negative pressure device. Since there is a certain distance between the negative pressure valve 500 and the negative pressure device, the connecting tube extending into the accommodating cavity 110 has a certain length. For this reason, for this portion of the connecting tube, this embodiment provides the first groove 161 on the wall of the accommodating cavity 110 to accommodate the connecting tube with a certain length, which avoids interference or intertwining of this portion of the connecting tube with the internal components of a handle of the endoscope, so that the internal components are arranged orderly and reasonably to ensure the stable operation of the endoscope.

Alternatively, the first groove 161 can be directly disposed into the wall of the accommodating cavity 110 by a grooving process, or can be protruded outward from the surface of the housing 100 and formed on the wall of the accommodating cavity 110.

In an embodiment, referring to FIGS. 30 and 31, the second valve body 510 includes a body section 514, a first branch section 515 and a second branch section 516. The first branch section 515 is connected to an end of the body section 514. The second branch section 516 is connected to one side of the body section 514. The second flow channel 511 is defined in the body section 514. The inlet 512 is defined on the first branch section 515, and the outlet 513 is defined on the second branch section 516. It can be seen from this that when drawing liquid or tissue, the second valve core 520 is driven to operate in the body section 514 so that the first branch section 515 and the second branch section 516 are in communication with each other through the third flow channel 521. In this case, the liquid or tissue flows through the inlet 512, the first branch section 515, the third flow channel 521 and the second branch section 516 in sequence, and be discharged from the outlet 513.

Specifically, referring to FIG. 30, the first branch section 515 and the second branch section 516 are both arranged at an angle with respect to the body section 514

Further, referring to FIG. 31, the second valve core 520 is movably sleeved in the body section 514. A first aperture 523 in communication with the third flow channel 521 is disposed at an end of the second valve core 520. The first aperture 523 is connected to the inlet 512. A second aperture 522 in communication with the third flow channel 521 is disposed on a side surface of the second valve core 520 attached to the inner wall of the second flow channel 511. By driving the second valve core 520, the second aperture 522 and the outlet 513 are misaligned with each other or correspond to each other. It can be seen from this that during switching between opening and closing of the channel, the second valve core 520 is driven to move in the second flow channel 511. When the second aperture 522 corresponds to the outlet 513, the outlet 513, the second aperture 522, the third flow channel 521, the first aperture 523 and the inlet 512 are in communication with one another, so that the channel in the negative pressure valve 500 is completely opened, and thus the negative pressure device can perform drawing to the inside of the organ. When the second aperture 522 and the outlet 513 are misaligned with each other, the outlet 513 and the inlet 512 cannot be in communication with each other, so that the channel in the negative pressure valve 500 is closed. In this case, the negative pressure device stops working.

In an embodiment, referring to FIGS. 27 and 30, the third coupling part 170 is a second slot 171, and the fourth coupling part 517 is a second insertion block 5171 that cooperates with the second slot 171. In this way, when fixing, the second insertion block 5171 is inserted into the second slot 171 so that the negative pressure valve 500 is stably mounted on the housing 100.

It should be noted that one, two or more the second slots 171 and one, two or more the second insertion blocks 5171 may be provided, and which is not specifically limited in this embodiment. In addition, there are many corresponding relationships between the second slots 171 and the second insertion blocks 5171, for example, the second insertion blocks 5171 are inserted into the second slots 171 in a one-to-one correspondence; or the second insertion blocks 5171 are inserted into the second slots 171 in a two-to-one correspondence, etc.

Certainly, in other embodiments, the third coupling part 170 may be the second insertion block 5171, and the fourth coupling part 517 is the second slot 171 that cooperates with the second slot 171.

Further, referring to FIG. 30, the second insertion block 5171 is extended on the second valve body 510 in the circumferential direction of the second valve body 510. This facilitates the second insertion block 5171 to be inserted into the second slot 171 in different orientations, so that the same second insertion block 5171 can be inserted into two or more second slots 171 at the same time.

In an embodiment, referring to FIG. 27, a third enclosure plate 172 and a fourth enclosure plate 173 are spaced apart on the wall of the accommodating cavity 110. The third enclosure plate 172 and the fourth enclosure plate 173 are respectively located on opposite sides of the third mounting hole 113 and enclose the second slot 171. In this way, the third enclosure plate 172 and the fourth enclosure plate 173 are respectively disposed on the opposite sides of the third mounting hole 113, so that when the negative pressure valve 500 extends through the third mounting hole 113, the second insertion block 5171 on the negative pressure valve 500 can just be inserted into the second slot 171, thereby making the mounting of the negative pressure valve 500 more convenient.

Optionally, the third enclosure plate 172 and the fourth enclosure plate 173 can be mounted on the housing 100 by means of, but not limited to, bolted connection, engaging, riveting, welding, bonding, integrally molding, etc. The integrally molding includes injection molding, 3D printing, etc.

In an embodiment, referring to FIGS. 23 and 26, the housing 100 includes the first housing 130 and the second housing 131. The first housing 130 covers the second housing 131 and the accommodating cavity 110 is enclosed by the first housing 130 and the second housing 131. The second slot 171 includes a third type of slot 1711 and a fourth type of slot 1712. The third type of slot 1711 is defined on the first housing 130. The fourth type of slot 1712 is defined on the second housing 131. An end of the second insertion block 5171 is inserted into the third type of slot 1711, and another end of the second insertion block 5171 is inserted into the fourth type of slot 1712. It can be seen from this that the housing 100 of this embodiment is composed of two parts. When the second housing 131 is fitted on the first housing 130, the opposite ends of the second insertion block 5171 are inserted into the third type of slot 1711 and the fourth type of slot 1712 respectively, so that the negative pressure valve 500 is more stable in the housing 100.

In an embodiment, referring to FIG. 30, the negative pressure valve 500 further includes a second valve cover 530 and a second elastic member 540. The second valve cover 530 is disposed on the second valve core 520. The second elastic member 540 is connected between the second valve cover 530 and the second valve body 510, and is configured to drive the second valve core 520 to block the inlet 512 and the outlet 513.

Optionally, the second elastic member 540 may be, but is not limited to, a spring, elastic rubber, elastic metal sheet, etc.

In an embodiment, referring to FIG. 30, the negative pressure valve 500 further includes a second valve sleeve 550. The second valve sleeve 550 is sleeved on the second valve core 520, and connected to the second valve body 510.

In an embodiment, referring to FIGS. 23 and 32, the endoscope further includes a three-way valve 600. The inner wall of the housing 100 is provided with a fifth coupling part 180. The three-way valve 600 is provided with a sixth coupling part 630 that is engaged with the fifth coupling part 180. The three-way valve 600 is provided with a first port 611, a second port 621 and a third port 612 that are in communication with one another. The first port 611 is in communication with the negative pressure valve 500. The second port 621 is configured to be in communication with a device channel valve 1000. The third port 612 is configured to be in communication with a device channel of the distal tip 900. In this way, compared with the conventional mounting method for the three-way valve 600, the method according to this embodiment does not require the use of bolts or screws and other components, thereby reducing the number of parts during assembling process of the endoscope and ensuring a simple and orderly assembling process.

It should be noted that one, two, three or more fifth coupling parts 180, and one, two, three or more sixth coupling parts 630 may be provided, and which is not specifically limited in this embodiment.

Alternatively, the fifth coupling part 180 may have a convex structure, and the sixth coupling part 630 may have a groove-like or hole-like structure; alternatively, the fifth coupling part 180 may have a groove-like or hole-like structure, and the sixth coupling part 630 may have a convex structure.

Specifically, referring to FIGS. 25 and 32, the fifth coupling part 180 is a third slot 181. The sixth coupling part 630 is a third insertion block 631 adapted to the third slot 181. That is, during mounting, the third insertion block 631 on the three-way valve 600 is directly inserted into the third slot 181 in the housing 100, so that the assembling can be completed, which greatly improves assembling efficiency and convenience.

In an embodiment, referring to FIG. 25, the wall of the accommodating cavity 110 is provided with a protruding base 118 configured to support the three-way valve 600. The third slot 181 is defined on the protruding base 118. In this way, while being inserted, the three-way valve 600 is also supported by the protruding base 118 to prevent the three-way valve 600 from being suspended in the housing 100, making the three-way valve 600 more stable in the housing 100, which is beneficial to improve the stability of the usage of product.

Optionally, the protruding base 118 may be mounted on the wall of the accommodating cavity 110 by means of, but not limited to, bolt connection, engaging, bonding, riveting, welding, integrally molding, etc. The integrally molding can include injection molding, 3D printing molding, etc.

Specifically, referring to FIG. 25, the protruding base 118 and the housing 100 are an integrally formed, that is, the protruding base 118 are formed by protruding from the wall of the accommodating cavity 110.

Further, referring to FIG. 25, a second groove 182 is disposed on a side surface of the protruding base 118 facing the three-way valve 600. The second groove 182 is configured to be adapted to the outer surface of the three-way valve 600. In this way, the second groove 182 cooperates with the outer surface of the three-way valve 600, so that the three-way valve 600 is more stable on the protruding base 118, which avoids the three-way valve 600 from shaking left and right during use.

It should be noted that "adapted to" should be construed as that: the groove shape of the second groove 182 should be consistent with the shape of the outer surface of the three-way valve 600, so that the outer surface of the three-way valve 600 can better fit into the second groove 182.

In an embodiment, referring to FIG. 25, the protruding base 118 is provided with a notch 183 in communication with the third slot 181. A connecting tube between the negative pressure valve 500 and the three-way valve 600 extends through the notch 183; alternatively, the three-way valve 600 extends through the notch 183. It can be seen from this that when the three-way valve 600 is inserted into the protruding base 118, there is a connecting tube between the three-way valve 600 and the negative pressure valve 500. To this end, the notch 183 is disposed on the protruding base 118 to facilitate the connecting pipe to extend out of the protruding base 118, which avoids structural interference between the connecting tube and the protruding base 118, and thus avoids difficulty in arranging the connecting tube. Certainly, during the mounting process, a portion of the three-way valve 600 can also extend out from the notch 183, which can also effectively avoid the problem of structural interference between the connecting tube and the protruding base 118, making the internal structural arrangement of the endoscope more reasonable. Certainly, in order to facilitate the distribution and fixation of the connecting tube, a tube bracket 114 can be disposed in the housing 100 to fix the connecting tubes and the pipelines on the gas-water valve 400.

In an embodiment, referring to FIG. 25, the protruding base 118 includes; a support plate 190; and a first side plate 191 and a second side plate 192 that are disposed on opposite sides of the support plate 190. The first side plate 191, the second side plate 192 and the support plate 190 enclose the third slot 181. The notch 183 is formed between the first side plate 191 and the second side plate 192. In this way, the structures of the support plate 190, the first side plate 191 and the second side plate 192 are utilized to simultaneously form the third slot 181 and the notch 183, which is beneficial to simplifying the structural design.

Specifically, referring to FIG. 25, the support plate 190, the first side plate 191 and the second side plate 192 are integrally formed.

In an embodiment, referring to FIGS. 23 and 26, the housing 100 includes the first housing 130 and the second housing 131. The first housing 130 covers the second housing 131 and the accommodating cavity 110 is enclosed by the first housing 130 and the second housing 131. The protruding base 118 includes a first protruding base 1181 and a second protruding base 1182. The first protruding base 1181 is disposed on the first housing 130. The second protruding base 1182 is provided on the second housing 131. The third slot 181 includes a fifth type of slot 1811 and a sixth type of slot 1812. The fifth type of slot 1811 is defined on the first protruding base 1181, and the sixth type of slot 1812 is defined on the second protruding base 1182. An end of the third insertion block 631 is inserted into the fifth type of slot 1811, and another end of the third insertion block 631 is inserted into the sixth type of slot 1812. It can be seen from this that when the first housing 130 and the second housing 131 cooperate with each other, the three-way valve 600 is respectively supported between the first protruding base 1181 and the second protruding base 1182; and in this case, the third insertion block 631 on the three-way valve 600 is respectively inserted into the fifth type of slot 1811 and the sixth type of slot 1812, thus making the mounting of the three-way valve 600 more stable.

It should be noted that, for ease of understanding and illustration, this embodiment divides the protruding base 118 into the first protruding base 1181 and the second protruding base 1182. Therefore, the first protruding base 1181, the second protruding base 1182 and the protruding base 118 are only different in name, but have the same or similar structure. Similarly, the fifth type of slot 1811 and the sixth type of slot 1812 are different in name from the third slot 181, but have similar structures to the third slot 181.

In an embodiment, referring to FIG. 25, two protruding bases 118 and two third insertion blocks 631 are provided. The two protruding bases 118 are spaced apart in the length direction of the housing 100. The two third insertion blocks 631 are spaced apart in the length direction of the three-way valve 600. The two protruding bases 118 correspond to the two third insertion blocks 631 one-by-one. The housing 100 is provided with a first opening 115 and a second opening 116 that are in communication with the accommodating cavity 110. The second port 621 extends through the first opening 115. The third port 612 is disposed toward the second opening 116. The notch 183 of one of the protruding bases 118 is disposed toward the negative pressure valve 500, and the notch 183 of the other of the protruding bases 118 is disposed toward the second opening 116.

In an embodiment, referring to FIG. 32, the three-way valve 600 includes a first tube 610 and a second tube 620. An end of the second tube 620 is connected to the second tube 620, and another end of the second tube 620 extends through the first opening 115. The first port 611 and the third port 612 are respectively disposed at opposite ends of the first tube 610. The second port 621 is disposed at the other end of the second tube 620 away from the first tube 610. The sixth coupling part 630 is disposed on the first tube 610 and/or the second tube 620. In this way, the three-way valve 600 has a good three-way control function.

The technical features of the above embodiments may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present application, and the description thereof is relatively specific and detailed, but it should not be construed as limiting the scope of the patent. It should be noted that for those skilled in the art, various modifications and improvements may be made without departing from the concept of the present application, and all these modifications and improvements belong to the protection scope of the present application. Therefore, the scope of protection of the patent application should be subject to the appended claims.

In the description of the present application, it should be understood that orientations or positional relationships indicated by the terms "center", "longitudinal", "transverse", "length", "width", "thickness", "upper", "lower", "front", "rear", "left", "right", "vertical", "horizontal", "top", "bottom", "inside", "outside", "clockwise", "counterclockwise", "axis", "radial", "circumferential", etc. are based on the orientations or positional relationships shown in the drawings, which are only for the convenience of describing the present application and simplifying the description, and do not indicate or imply the device or element indicated must have a specific orientation, be constructed and operate in a specific orientation and therefore are not to be construed as limitations of the present application.

In addition, the terms "first" and "second" are used for descriptive purposes only and cannot be understood as indicating or implying relative importance or implicitly indicating the quantity of indicated technical features. Therefore, features defined by "first" and "second" may explicitly or implicitly include at least one of these features. In the description of the present application, "plurality" means at least two, such as two, three, etc., unless otherwise expressly and specifically limited.

In the present application, unless otherwise clearly stated and limited, the terms "installation", "coupling", "connecting", "fixing" and the like should be understood in a broad sense, and for example, may be a fixed connection or a detachable connection., or integrally formed; or may be a mechanical connection or an electrical connection; or may be a direct connection or an indirect connection through an intermediate medium; or may be an internal communication between two elements or an interactive relationship between two elements, unless otherwise specified defined. For those of ordinary skill in the art, the specific meanings of the above terms in the present application can be understood according to specific circumstances.

In the present application, unless otherwise expressly stated and defined, a first feature being "on" or "below" a second feature may mean that the first feature is in direct contact with the second feature, or the first feature is in indirect contact with the second feature through an intermediate medium. Furthermore, a first feature being "above", "over" or "on top of' a second feature may mean that the first feature is directly above or diagonally above the second feature, or simply means that the first feature is higher in level than the second feature. A first feature being "below", "under" and "beneath" a second feature may mean that the first feature is directly below or diagonally below the second feature, or simply means that the first feature higher in level than the second feature.

It should be noted that when an element is referred to as being "fixed on" or "disposed on" another element, it can be directly on the other element or an intermediate element may also be present. When an element is referred to as being "connected to" another element, it can be directly connected to the other element or there may also be intermediate element. The terms "vertical", "horizontal", "upper", "lower", "left", "right" and similar expressions used herein are for illustrative purposes only and do not represent the unique implementations.

## Claims

1. An endoscope, comprising:
a housing provided with a first mounting hole that is in communication with inside and outside of the housing;
a knob device comprising a first pulley, a second pulley, a first knob and a second knob, the first pulley and the second pulley being rotatably mounted in the housing, the first knob and the second knob being located outside the housing, the first pulley extending out of the housing through the first mounting hole, and wherein a first through hole extends through the first pulley, the second pulley extends out of the housing through the first through hole, and wherein the first knob is sleeved on the first pulley, and the second knob is stacked on the first knob and sleeved on the second pulley;
a braking device comprising a first braking assembly and a second braking assembly, and wherein the first braking assembly is disposed between the first pulley and the housing, and configured to brake the first knob or the first pulley, and wherein the second braking assembly is disposed on a side of the second knob facing away from the first knob, and configured to brake the second knob or the second pulley; and
a bending section located outside the housing, and respectively connected to the first pulley and the second pulley through a pulling wire.

2. The endoscope according to claim 1, wherein the first braking assembly comprises:
a first fixed frame;
a first braking button; and
at least two first braking members slidably disposed on the first fixed frame,
wherein the first fixed frame is positioned on the housing; the first braking button is sleeved outside the first fixed frame; and when the first braking button is rotated, the first braking button is configured to push the first braking member to slide to hold the first knob or the first pulley tightly.

3. The endoscope according to claim 2, wherein the first fixed frame is provided with a first working chamber into which the first pulley extends; the first knob extends into the first working chamber and is sleeved on the first pulley; and when the first braking button rotates, the first braking button is configured to push the first braking member to extend into the first working chamber and hold the first knob tightly.

4. The endoscope according to claim 1, wherein the knob device further comprises a first bracket disposed in the housing; a second through hole extends through the second pulley; the first bracket extends through the second through hole and extends out of the housing; and the second braking assembly is mounted on the first bracket.

5. The endoscope according to claim 4, wherein the second braking assembly comprises:
a second fixed frame;
a second braking button; and
at least two second braking members slidably disposed on the second fixed frame,
wherein the second fixed frame is positioned on the first bracket; the second braking button is sleeved outside the second fixed frame; and when the second braking button rotates, the second braking button is configured to push the second braking member to slide to hold the second knob or the second pulley tightly.

6. The endoscope according to claim 5, wherein the first bracket is provided with a first fixing hole extending therethrough; the second braking button is provided with a second fixing hole corresponding to the first fixing hole; a fastener is inserted into the first fixing hole and the second fixing hole, so that the second braking button is rotatably mounted on the first bracket.

7. The endoscope according to claim 5, wherein the second knob is provided with a braking ring; the second fixed frame is provided with a second working chamber into which the braking ring extends; and when the second braking button rotates, the second braking button is configured to push the second braking member to extend into the second working chamber and hold the brake ring tightly.

8. The endoscope according to any one of claims 1 to 7, further comprising:
a gas-water valve; and
a distal tip disposed on the bending section,
wherein the gas-water valve is embedded in the housing; a first coupling part is disposed on an inner wall of the housing; the gas-water valve is provided with a second coupling part that is engaged with the first coupling part.

9. The endoscope according to any one of claims 1 to 7, further comprising a negative pressure valve embedded in the housing,
wherein a third coupling part is disposed on an inner wall of the housing; and the negative pressure valve is provided with a fourth coupling part that is engaged with the third coupling part.

10. The endoscope according to claim 9, further comprising a three-way valve,
wherein the inner wall of the housing is provided with a fifth coupling part; the three-way valve is provided with a sixth coupling part that is engaged with the fifth coupling part; the three-way valve is provided with a first port, a second port and a third port that are in communication with one another; the first port is in communication with the negative pressure valve; the second port is configured to be in communication with a device channel valve; and the third port is configured to be in communication with a device channel of the distal tip.
